# EUROPEAN PATENT APPLICATION

(11) **EP 1 595 548 A2**
(43) Date of publication of application: **16.11.2005**
(21) Application number: 05014175.3
(22) Date of filing: 07.03.2002
(51) Int. Cl.: A61K 39/00

(54) **Anti-neovasculature preparations for treating cancer**

(30) Priority: 07.03.2001 US 274063 P
(62) Divisional of application: 02715085.3
(71) Applicant: Mannkind Corporation, Chatsworth, California 91311 (US)
(72) Inventor: Simard, John J.L., Northridge, CA 91326 (US); Diamond, David C., West Hills, CA 91304 (US)
(74) Representative: Neuefeind, Regina

(57) **Abstract**

Disclosed herein are immunogenic compositions, methods of designing immunogenic compositions, methods of treatment using immunogenic compositions, methods of evaluating cell-mediated immunity resulting from immunogenic compositions, research models, and methods of making research models, all of which relate to targeting tumor vasculature.

## Description

Disclosed herein are immunogenic compositions, methods of designing immunogenic compositions, methods of treatment using immunogenic compositions, methods of evaluating cell-mediated immunity resulting from immunogenic compositions, research models, and methods of making research models, all of which relate to targeting tumor vasculature.

The treatment of cancer has remained challenging despite the advances in biomedicine. In recent years two approaches have been described showing much promise: therapeutic vaccines and anti-angiogenesis.

Therapeutic vaccines rely on the observation that cancerous tissues generally express certain antigens preferentially, collectively tumor-associated antigens (TuAA). TuAA include proteins normally expressed selectively by the tissue from which the cancer derives (differentiation antigens), proteins that are associated with a different stage of development (oncofetal and cancer-testis antigens), proteins that are created by aberrant chromosomal rearrangement, or proteins that are derived from oncogenic viruses. These TuAA, or fragments of them, are then used as immunogens in vaccines intended to stimulate cellular immunity, particularly cytotoxic T lymphocytes (CTL), capable of killing the tumor cells.

The anti-angiogenesis approach takes advantage of the need of tumors to recruit a blood supply to support their continued growth. To accomplish this, tumors secrete angiogenic factors that promote the growth of new blood vessels. The anti-angiogenesis approach aims to disrupt a tumor's supply of nutrients to cause it to die, or at least limit its growth. Attempts at this approach have sought chemotherapeutic drugs used directly against a variety of anti-angiogenic factors and angiogenesis.

The invention disclosed herein is directed to compositions designed to stimulate cellular immune responses targeting tumor-associated neovasculature (TuNV). In one embodiment of the invention the compositions stimulate a CTL response. Such compositions may include one or more epitopes of the target antigen. One aspect of this embodiment specifically includes a housekeeping epitope, another specifically includes an immune epitope or epitope cluster, and another aspect specifically combines housekeeping and immune epitopes.

Embodiments of the invention relate to the use of prostate specific membrane antigen (PSMA) as the target antigen of the composition. Aspects of this embodiment include various epitopes derived from PSMA provided directly as polypeptide, or as a nucleic acid capable of conferring expression of the epitope. Other embodiments relate to the use of other TuNV-associated antigens.

In other embodiments of the invention, compositions are directed against both the TuNV and against TuAA expressed by the cancerous cells, by combining immunogens derived from both sources into a single formulation or method or treatment.

Preclinical evaluation of the compositions of this invention can be accomplished using adoptive transfer of immunized T cells into SCID mice bearing microvasculature formed from implanted human dermal microvascular endothelial cells (HDMEC). Preclinical evaluation can also be accomplished through the use of HLA-transgenic mice immunized with compositions comprised of epitopes conserved between mice and humans.

Embodiments of the invention relate to methods of evaluating cell-mediated immunity. The methods can include the steps of implanting vascular cells into an immunodeficient mammal; establishing an immune response in the mammal; and assaying a characteristic to determine cell-mediated immunity in the mammal. The cell-mediated immunity can be directed against a neovasculature antigen, for example. The neovasculature antigen can be preferentially expressed by tumor-associated neovasculature, for example, and in preferred embodiments can be prostate specific membrane antigen (PSMA), vascular endothelial growth factor receptor 2 (VEGFR2), and the like. The establishing step can be achieved, for example, by adoptive transfer of T-cells to the mammal, by contacting the mammal with an antigen, and the like. The cell-mediated immunity can be mediated by cytotoxic T lymphocytes. The vascular cells can be vascular endothelial cells, such as, for example, human dermal microvascular endothelial cells (HDMEC), telomerase-transformed endothelial cells, and the like. The immunodeficient mammal can be a mouse, such as for example a SCID mouse. The characterizing step can include assessing a parameter, such as for example, vessel formation, vessel destruction, vessel density, proportion of vessels carrying blood of the host mammal, and the like.

The methods can further include the step of implanting tumor cells or tumor tissue in the mouse. The characterizing step can include assessing a parameter, such as, for example, tumor presence, tumor growth, tumor size, rapidity of tumor appearance, dose of vaccine required to inhibit or prevent tumor establishment, tumor vascularization, a proportion of necrotic tissue within the tumor, and the like.

The methods can further include the steps of providing a first population of mammals and a second populations of mammals; establishing cell-mediated immunity in the first population; differentially establishing cell-mediated immunity in the second population; and comparing a result obtained from the first population of mammals to a result obtained from the second population of mammals. The cell-mediated immunity of the first population can include, for example, naïve immunity, immunity to an irrelevant epitope, and the like.

Other embodiments relate to methods of evaluating cell-mediated immunity, including immunity directed against a neovasculature antigen. The methods can include the steps of implanting or injecting MHC-transgenic tumor cells into an MHC-transgenic mammal; establishing an immune response in the mammal; and assaying a characteristic to determine cell-mediated immunity in the mammal. The MHC-transgenic mammal can be an HLA-transgenic mammal, such as, for example an HLA-A2 transgenic mammal. In preferred embodiments the mammal can be a mouse. The cell-mediated immunity can be established by vaccination, which in preferred embodiments can take place prior to, concurrent with, or subsequent to transfer of the tumor cells, for example. In preferred embodiments the cell-mediated immunity can be mediated by cytotoxic T lymphocytes. The neovasculature antigen can be preferentially expressed by tumor-associated neovasculature and can also be a tumor-associated antigen. Preferably, the antigen can be the ED-B domain of fibronectin. The characterizing step can include, for example, assessing a parameter, including tumor presence, tumor growth, tumor size, rapidity of tumor appearance, dose of vaccine required to inhibit or prevent tumor establishment, tumor vascularization, a proportion of necrotic tissue within the tumor, and the like. The methods can further include the steps of providing a first population of mammals and a second populations of mammals; establishing cell-mediated immunity in the first population; differentially establishing cell-mediated immunity in the second population; and comparing a result obtained from the first population of mammals to a result obtained from the second population of mammals. The cell-mediated immunity of the first population can include naive immunity, immunity to an irrelevant epitope, and the like.

Still further embodiments relate to methods of treating neoplastic disease, including the step of immunizing a mammal to induce a cellular immune response directed against an antigen differentially expressed by tumor-associated neovasculature. The differentially expressed antigen can be a protein, such as, for example prostate specific membrane antigen, vascular endothelial growth factor receptor 2 (VEGFR2), and the like. In other preferred embodiments, the antigen can be the ED-B domain of fibronectin. The immunization can be carried out, for example, with at least one peptide derived from the sequence of the protein, with a nucleic acid capable of conferring expression of the protein or peptides, and the like. The at least one peptide can include a housekeeping epitope, for example, and in preferred embodiments can be co-C-terminal with the housekeeping epitope. The methods can further include at least one additional peptide, wherein the at least one additional peptide includes an immune epitope. The methods can include an additional step wherein the mammal is treated with an anti-tumor therapy active directly against cancerous cells. The anti-tumor therapy can be immunization against a tumor-associated antigen. Preferably, the cellular immune response can include a CTL response.

Other embodiments relate to immunogenic compositions. The immunogenic compositions can include at least one immunogen corresponding to an antigen expressed by tumor-associated neovasculature, wherein the composition can induce a cellular immune response. The immunogen can be one that is not associated with a cell conspecific with the recipient. The antigen can be a protein, such as, for example prostate specific membrane antigen, vascular endothelial growth factor receptor 2 (VEGFR2), and the like. In other preferred embodiments the antigen can be the ED-B domain of fibronectin. The immunogen can include at least one peptide. The compositions can include a nucleic acid capable of conferring expression of the antigen, and wherein the antigen is a protein or a peptide. The compositions can include at least one peptide that includes a housekeeping epitope, and in preferred embodiments the at least one peptide can be co-C-terminal with the housekeeping epitope. Also, the compositions can additionally include at least one peptide that includes an immune epitope. The compositions can include at least one immunogen corresponding to a tumor-associated antigen. In preferred embodiments the cellular immune response can include a CTL response.

Embodiments relate to methods of anti-tumor vaccine design. The methods can include the steps of identifying an antigen differentially expressed by tumor-associated neovasculature; and incorporating a component of the antigen into a vaccine. The component can include, for example, a polypeptide fragment of the antigen, a nucleic acid encoding the antigen or a fragment of the antigen, and the like.

Further embodiments relate to methods of making a research model. The methods can include implanting a vascular cell and a tumor cell into an immunodeficient mammal. The tumor cell and the vascular cell can be implanted adjacent to one another. The vascular cell can be a vascular endothelial cell, such as for example HDMEC. In preferred embodiments the vascular endothelial cell can be telomerase-transformed. The immunodeficient mammal can be a mouse, such as, for example, a SCID mouse.

Other embodiments relate to research models. The research models can include an immunodeficient mammal. The mammal can include an implanted vascular cell and an implanted tumor cell. The vascular cell and the tumor cell can be implanted adjacent to one another.

### Detailed Description of the Preferred Embodiment

Embodiments of the invention disclosed herein provide compositions, methods of composition or vaccine design, and methods of treatment related to the generation of a cellular immune response, preferably, a T cell response and, more preferably, a CTL response, directed against the neovasculature of tumors. Such methods and compositions are particularly useful in the treatment and prevention of cancer. Other embodiments relate to composition evaluation models.

### Compositions, Composition Design, and Treatment Using the Compositions

Embodiments of the invention relate to immunogenic compositions, including vaccines, for the generation of a cellular immune response, particularly a T cell response and specifically a CTL response, directed against tumor neovasculature (TuNV). "Tumor neovasculature" is broadly meant to include any vasculature found in or around tumor masses, vasculature which supports or is necessary for tumor growth, and the like. It should be noted, and one of skill in the art will appreciate, that although the discussion herein refers generally to the tumors and tumor neovasculature, the embodiments of the present invention also can be applied to other conditions or disease states associated with inappropriate angiogenesis.

Until now the design of anti-tumor vaccines has concentrated on antigens expressed by the malignant cells themselves. However, larger tumors are complex structures and not simply a homogeneous mass of cells. All cells, particularly rapidly growing cells, need a supply of nutrients (oxygen, glucose, amino acids, etc.), as well as a means of removal of metabolic wastes, in order to remain metabolically active and intact. This is normally accomplished by the flow of blood and lymph through the various organs of the body. At a cellular level, the tissues of the body are permeated by a fine network of capillaries - tiny vessels through which nutrients and waste products can be exchanged with the surrounding cells by diffusion. Diffusion is effective over relatively short distances. The capillary beds are so extensive that generally cells are at most located only a few cells away from a capillary. If a tumor merely grew by propagation of its malignant cells, soon those cells in the interior of the mass would be unable to sustain themselves. In fact, the interiors of unvascularized tumors often contain necrotic tissue. Thus, in order to grow unchecked, tumors secrete factors that promote the ingrowth of new blood vessels, namely TuNV. Since the TuNV expresses antigens differentiating it from other tissues, cancer can be treated with therapeutic compositions directed against the TuNV, instead of directly targeting the cancerous cells themselves. Suitable TuNV antigens can include those that are expressed generally in neovasculature or preferentially by TuNV, for example.

In some embodiments of the invention the compositions can include, for example, an epitopic peptide or peptides. Immune epitopes may be provided embedded in epitope clusters and protein fragments. Housekeeping epitopes can be provided with the proper C-terminus. In other embodiments of the invention the compositions can include nucleic acids capable of conferring expression of these epitopes on pAPC, for example.

In preferred embodiments, the compositions can be administered directly to the lymphatic system of a mammal being treated. This can be applied to both polypeptide and nucleic acid based compositions. Administration methods of this type, and related technologies, are disclosed in U.S. Patent Application No. 09/380,534, filed on September 1, 1999, and a Continuation-in-Part thereof, filed on February 2, 2001; U.S. Patent Application No. 09/776,232, both entitled "A METHOD OF INDUCING A CTL RESPONSE,".

In a preferred embodiment, destruction of the blood vessels in a tumor by action of a composition of the invention can eliminate *all* of the cells in a tumor. However, small tumors, including micrometastases, are typically unvasculaturized. Additionally, unvascularized tumors that instead apparently rely on blood flow through channels penetrating the tumor mass have been reported (Maniotis, A.J., et al. Am. J. Pathol. 155: 739-752, 1999). Thus in other embodiments, the compositions are generally effective as tumor control agents that may not eradicate all cancer cells. Accordingly, the invention provides tools for eliminating tumors, controlling tumor growth, reducing tumor burden, improving overall clinical status, and the like. In some embodiments, it can be desirable to combine these compositions with other treatments that target the cancerous cells directly. Additionally there is evidence that the vasculature in tumors can be mosaic in nature consisting of both endothelial and cancer cells (Chang, Y.S., et al. Proc. Natl. Acad. Sci. USA 97:14608-14613, 2000). Thus, in some embodiments of the invention a course of composition treatment can be followed by administration of a bio- or chemotherapeutic agent. In a particularly preferred embodiment, treatment can include administration of a TuAA directed composition concurrent or subsequent to administration of the anti-TuNV composition.

As mentioned above, suitable TuNV antigens for the compositions can include those that are expressed generally in neovasculature or preferentially by TuNV, for example. A variety of techniques for discovery of TuAA are known in the art. Examples of these techniques include, without limitation, differential hybridization and subtractive hybridization, including use of microarrays; expression cloning; SAGE (serial analysis of gene expression); SEREX (serological identification of antigens by recombinant expression cloning); in situ RT-PCT; immunohistochemistry (as was the case for PSMA); EST analysis; variously using bulk, sectioned, and/or microdissected tissue; and the like. Utilization of these and other methods provides one of skill in the art the techniques necessary to identify genes and gene products contained within a target cell that may be used as antigens of immunogenic compositions. The techniques are applicable to TuAA discovery regardless of whether the target cell is a cancer cell or an endothelial cell. Any identified antigen can be scrutinized for epitopes, which can be used in embodiments of the invention.

The endothelial cells making up the lining of the vasculature can express housekeeping proteasomes. Thus, compositions targeting endothelial cells can be comprised of peptides, or nucleic acids conferring expression of the peptides, corresponding to the digestion products of the housekeeping proteasome (i.e. housekeeping epitopes). IFN-γ (gamma), secreted by activated cells of the immune system, can induce expression of the immunoproteasome in the target cells. Generally, the immunoproteasome is constitutive in professional antigen presenting cells (pAPC). Thus, it can be helpful to include immune epitopes or epitope clusters in CTL-inducing compositions to ensure that there are CTL able to recognize the target cell regardless of the state that the target cell is in. This can be particularly true with endothelial cells, which readily assume antigen presentation functions. These concepts are more fully explained in U.S. Patent Application Nos. 09/560,465, filed on April, 28, 2000; 10/005,905, filed on November 7, 2001; and a continuation thereof, U.S. Application No. filed on December 7, 2001, attorney docket number CTLIMM.21CP1C, each of which is entitled "EPITOPE SYNCHRONIZATION IN ANTIGEN PRESENTING CELLS,".

As discussed above, the immunogenic compositions, including in preferred embodiments, vaccines, can include TuNV antigens and epitopes, for example. The epitopes can include one or more housekeeping epitopes and/or one or more immune epitopes. Specific epitopes useful in compositions can be identified using the methods disclosed in U.S. Patent Application No. 09/561,074 entitled "METHOD OF EPITOPE DISCOVERY," filed on April 28, 2000. For example, peptide sequences that are known or predicted to bind to some MHC restriction element can be compared to fragments produced by proteasomal digestion in order to identify those that are co-C-terminal.

Examples of useful epitopes for the embodiments of the invention, including epitopes of ED-B and PSMA, are disclosed in a U.S. Provisional Patent Application No. , entitled "EPITOPE SEQUENCES," attorney docket number CTLIMM.027PR, filed on even date with this application, March 7, 2002, and two U.S. Provisional Patent Applications, each entitled "EPITOPE SEQUENCES;" Application No. 60/282,211, filed on April 6, 2001 and 60/337,017, filed on November 7, 2001. Each of these applications is incorporated herein by reference in its entirety.

PSMA is one example of a TuAA that can be targeted in some embodiments. PSMA is expressed in the neovasculature of most tumor types, but not by the vascular endothelium of normal tissues (Chang, S.M. et al., *Cancer Res.* 59(13):3192-8,1999; *Clin Cancer Res.* 10:2674-81, 1999). PSMA is a membrane antigen, and as such, it may be possible to attack PSMA-expressing TuNV with monoclonal antibody (mAb). However, the effectiveness of mAb in the treatment of cancer has proved to be more difficult than initially anticipated. Moreover, as other antigens are discovered to be associated with the TuNV, it is likely that many of them will prove not to be expressed at the vasculature surface, making them inaccessible to mAb attack.

T cells, particularly CTL, on the other hand, survey the expression of internal components of the cell through the process of major histocompatability complex (MHC)-restricted antigen presentation. The parameters for determining the effectiveness of T cell-activating vaccines and compositions against self-antigens are subtle. Some of the critical features and parameters relating to appropriate epitope selection are disclosed in U.S. Patent Application No. 09/560,465 entitled "EPITOPE SYNCHRONIZATION IN ANTIGEN PRESENTING CELLS," filed on April 28, 2001; U.S. Patent Application No. 09/561,074 entitled "METHOD OF EPITOPE DISCOVERY," filed on April 28, 2001; and U.S. Patent Application No. 09/561,571 entitled "EPITOPE CLUSTERS," filed on April 28, 2001. Features of DNA vaccine design promoting epitope synchronization are disclosed in U.S.

Patent Application No. 09/561,572 entitled "EXPRESSION VECTORS ENCODING EPITOPES OF TARGET-ASSOCIATED ANTIGENS," filed on April 28, 2001 and U.S. Provisional Application No. 60/336,968 entitled "EXPRESSION VECTORS ENCODING EPITOPES OF TARGET-ASSOCIATED ANTIGENS AND METHODS FOR THEIR DESIGN," filed on November 7, 2001. Particularly effective means of vaccine delivery are described in U.S. Patent Application No. 09/380,534, filed on September 1, 1999, and a Continuation-in-Part thereof, U.S. Patent Application No. 09/776,232, filed on February 2, 2001, both entitled "A METHOD OF INDUCING A CTL RESPONSE." Each of the above-mentioned references is incorporated herein by reference in its entirety.

Another example of a TuNV antigen that can be used in embodiments is fibronectin, preferably the ED-B domain. Fibronectin is subject to developmentally regulated alternative splicing, with the ED-B domain being encoded by a single exon that is used primarily in oncofetal tissues (Matsuura, H. and S. Hakomori *Proc. Natl. Acad. Sci. USA* 82:6517-6521, 1985; Carnemolla, B. et al. *J. Cell Biol*. 108:1139-1148, 1989; Loridon-Rosa, B. et al. *Cancer Res.*50:1608-1612, 1990; Nicolo, G. et al. *Cell Differ. Dev.* 32:401-408, 1990; Borsi, L. et al. *Exp. Cell Res*. 199:98-105, 1992; Oyama, F. et al. *Cancer Res.* 53:2005-2011, 1993; Mandel, U. et al. *APMIS* 102:695-702, 1994; Farnoud, M.R. et al. *Int. J. Cancer* 61:27-34, 1995; Pujuguet, P. et al. Am. J. Pathol. 148:579-592, 1996; Gabler, U. et al. *Heart* 75:358-362, 1996; Chevalier, X. *Br. J. Rheumatol.* 35:407-415, 1996; Midulla, M. *Cancer Res.* 60:164-169,2000).

The ED-B domain is also expressed in fibronectin of the neovasculature (Kaczmarek, J. et al. *Int. J. Cancer* 59:11-16, 1994; Castellani, P. et al. *Int. J Cancer* 59:612-618, 1994; Neri, D. et al. *Nat. Biotech.* 15:1271-1275, 1997; Karelina, T.V. and A.Z. Eisen *Cancer Detect. Prev.* 22:438-444, 1998; Tarli, L. et al. *Blood* 94:192-198, 1999; Castellani, P. et al. *Acta Neurochir. (Wien)* 142:277-282, 2000). As an oncofetal domain, the ED-B domain is commonly found in the fibronectin expressed by neoplastic cells, in addition to being expressed by the TuNV. Therefore, CTL-inducing compositions targeting the ED-B domain can exhibit two mechanisms of action: direct lysis of tumor cells, and disruption of tumor blood supply through destruction of the TuNV.

It should be noted that expression of the fibronectin ED-B domain has been reported in both tumor-associated and normal neovasculature (Castellani, P. et al. *Int. J. Cancer* 59:612-618, 1994). Thus, compositions based on it, or similarly expressed antigens, can be effective against other conditions associated with inappropriate angiogenesis. Further, as CTL activity can decay rapidly after withdrawal of the composition, interference with normal angiogenesis can be minimal.

Other targets for the immunogenic compositions include growth factor receptors, including those associated with vascular cells. One such example is the vascular endothelial growth factor receptor 2 (VEGFR2). U.S. Patent No. 6,342,221 includes discussion of VEGF and VEGFR2. One of skill in the art will appreciate that any other antigen or protein associated with vascular cells can be a target for the immunogenic compositions, including those that are presently known and those yet to be identified.

### Animal Models, Methods of Making the Models, and Composition Evaluation

Compositions designed based upon the preceding considerations are effective against the various targets. However, additional evaluation can be easily performed at any time, but preferably in a pre-clinical setting. For example, such evaluation can be used in order to further aid in composition design. Other embodiments of the invention relate to methods of evaluating the immunogenic compositions. The compositions of the present invention can be easily evaluated by one of skill in the art using animal models for composition evaluation. For example, following the routine procedures below, one of skill in the art can evaluate TuNV compositions quickly and efficiently. Thus, using the models or guidance described herein, one of skill in the art can evaluate any TuNV composition for any TuNV antigen with little or no experimentation. Further embodiments relate to methods of making the animal research models. Other embodiments relate to the research model animals. These embodiments are set forth more fully below.

### Xenotransplanted Human Vasculature-Based Model

Some embodiments relate to a model system for studying the mechanisms of human microvessel formation. For example, in some embodiments, the model system can be used for preclinical evaluation of compositions. The model involves the subcutaneous implantation of telomerase-transformed human dermal microvascular endothelial cells (HDMEC) mixed with MATRIGEL (Becton Dickinson) into SCID mice. Subcutaneous implantation of telomerase-transformed HCMEC is described in Yang, J. et al. Nature Biotech 19:219-224, 2001. T cells activated by the compositions of this invention can be adoptively transferred, for example, into such implanted mice, and the ability of the T cells to destroy, or prevent the formation of, such human microvessels can be assessed. In other embodiments, the mouse can be directly vaccinated and evaluated. Also, in still further embodiments, the model system can be further adapted for testing compositions effective in non-human species by substituting DMEC from other species and species-matched telomerase, and by using analogous reagents to those described below for the human system.

The MHC-restriction elements presenting the epitopes of the composition being tested, preferably, are shared by the HDMEC line implanted into the mice. The T cells can be derived from *in vitro* immunization of human T cells, or by immunization of HLA-transgenic mice (procedures for which are well known in the art and examples of which are provided in the above incorporated patent applications). Use of T cells generated in HLA-transgeneic mice allows matching of genetic backgrounds between the adoptively transferred T cells and the host, thereby reducing the possibility of allogeneic or xenogeneic reactions that might complicate interpretation of the results. However, depending on the mouse strains available, this might require cross-breeding to get the HLA-transgene and SCID phenotype on the same genetic background. In the alternative, the donor T cells (human or murine) can be subjected to one or more rounds of *in vitro* stimulation to enrich for the desired population or establish a clone, and thereby similarly avoid undesired reactivities.

Techniques for *in vitro* immunization are know in the art, for example, Stauss et al., *Proc. Natl. Acad. Sci. USA* 89:7871-7875, 1992; Salgaller et al. *Cancer Res.* 55:4972-4979, 1995; Tsai et al., *J. Immunol*. 158:1796-1802, 1997; and Chung et al., *J. Immunother.* 22:279-287, 1999. Once generated, whether *in vivo* or *in vitro*, sufficient numbers of such T cells can be obtained by expansion *in vitro* through stimulation with the compositions of this invention and/or cytokines (see for example Kurokawa, T. et al., *Int. J. Cancer* 91:749-746, 2001) or other mitogens. These T cells can constitute a clone or a polyclonal population recognizing one or more epitopes. In preferred embodiments, on the order of 10⁵ to 10⁸ cells are transferred for adoptive transfer experiments in mice (see for example Drobyski, W.R. et al. *Blood* 97:2506-2513, 2001; Seeley B.M. et al. *Otolaryngol. Head Neck Surg*. 124:436-441, 2001; Kanwar, J.R. et al. *Cancer Res.* 61:1948-1956, 2001). Clones and otherwise more enriched populations generally require the transfer of fewer cells.

Transfer of the T cells can take place shortly before, concurrent with, or subsequent to implantation or establishment of the HDMEC. Parameters that can be assessed to evaluate effectiveness of the compositions include vessel formation, changes in vessel density, and ability to carry mouse blood (as described in Yang et al.), and the like. Assessment can be carried out as early as one week, and at least as long as 6 weeks, after implantation of telomerase-transformed HDMEC, preferably after 2 weeks; and from a day to more than 6 weeks after T cell transfer, preferably after 1 to 3 weeks. Generally, assessment can include comparison of mice receiving T cells reactive with the target antigen with mice receiving naïve (including sham-immunized), or irrelevant epitope-reactive T cells.

Relevant antigens can be expressed generally in neovasculature or preferentially by TuNV. Expression can be confirmed by a variety of techniques known in the art, including immunohistochemistry and RT-PCR. For example, tumor cells can be implanted along with the HDMEC. This can result in inducing expression of antigens preferentially expressed by TuNV. In one example, this can be accomplished by implanting a block of tumor tissue adjacent to the HDMEC-containing MATRIGEL implant, injecting tumor cells at the site of the implant, implanting tumor cell-containing MATRIGEL adjacent to the HDMEC-containing MATRIGEL implant, incorporating both tumor cells and HDMEC into the same MATRIGEL implant or by any other suitable method. As discussed above, in some embodiments, tumor cells can be implanted along with vascular cells. The animals so made, can be used as research models. Additional variations will be apparent to one of skill in the art.

### HLA-transgenic Animal Model

For antigens that are conserved, in sequence and/or expression profile, between human and the model species, HLA-transgenic strains allow another approach, namely vaccination of the model animal to combat a syngeneic tumor. The ED-B domain of fibronectin provides such an opportunity, as it is a marker of angiogenesis and has identical amino acid sequence in both humans and mice (Nilsson, F. et al. *Cancer Res.* 61:711-716, 2001). Moreover, spontaneous tumor tissue from a strain of HLA-A2 transgenic mice has been isolated and propagated. Epitope discovery and selection, and composition design and delivery for CTL inducing compositions are discussed above.

The tumor cell line, M1, is derived from a spontaneous salivary glandular cystadenocarcinoma. The M1 tumor cell line and methods of using the same is disclosed in U.S. Provisional Application No. , attorney docket number CTLIMM.028PR, filed on even date with this application, March 7, 2001, entitled "AN HLA-TRANSGENIC MURINE TUMOR CELL LINE." The tumor cell line, can arise in individuals of the HHD-A2 transgenic mouse strain of S. Pascolo et al. (J. Exp. Med. 185:2043-2051, 1997). These mice express a single monochain class I molecule comprising human β (beta)2-microglobulin, and α1 (alpha-1), and α2 (alpha-2) domains of HLA-A2.1 with the balance of the molecule derived from the murine class I molecule H2D^{b}. Blocks of tumor can be transplanted into new individuals where the tumor will re-grow, commonly within 1-3 weeks, with 3 mm blocks growing to 3 cm. Alternatively, tumor tissue can be disaggregated and the tumor cells grown *in vitro.* Upon harvest, the tumor cells can be injected subcutaneously into the neck or abdomen (2.5x10⁶ cells for 1-3 successive days), to result in a visible tumor in approximately 5-12 weeks for early passage cells. After the cells have become better adapted to growth *in vitro,* single injections of 1x10⁶ to 1x10⁷ cells lead to visible tumor in ten days. Generally, the initial tumor consistently occurs in the vicinity of the salivary glands, but secondary tumors can also occur in a variety of locations, including kidney, lung, liver, and abdominal muscle.

To evaluate the efficacy of a composition, it can be administered before, concurrent with, or subsequent to establishment of the tumor, depending on the expected mechanism of the composition. For therapeutic compositions intended to be used with some sort of debulking technique (e.g. surgery), concurrent administration can be appropriate. The better established the tumor is before treatment begins, the more stringent the test.

Both animal evaluation models have been described for the testing of human compositions. However, application to veterinary compositions is analogous, requiring only the substitution of species-matched endothelial cells, MHC, TuAA, etc.

The following examples are intended for illustration purposes only, and should not be construed as limiting the scope of the invention in any way.

### EXAMPLES

### Example 1.

A preclinical study was carried out using the already identified antigens PSMA and ED-B disclosed herein. The results of the study revealed excellent candidate epitopes. See table 9 below.

### Example 1.1

### Cluster Analysis (PSMA₁₆₃₋₁₉₂)

A peptide, AFSPQGMPEGDLVYVNYARTEDFFKLERDM, PSMA₁₆₃₋₁₉₂, (SEQ ID NO. 3), containing an A1 epitope cluster from prostate specific membrane antigen, PSMA₁₆₈₋₁₉₀ (SEQ ID NO. 4) was synthesized using standard solid-phase F-moc chemistry on a 433A ABI Peptide synthesizer. After side chain deprotection and cleavage from the resin, peptide first dissolved in formic acid and then diluted into 30% Acetic acid, was run on a reverse-phase preparative HPLC C4 column at following conditions: linear AB gradient (5% B/min) at a flow rate of 4 ml/min, where eluent A is 0.1 % aqueous TFA and eluent B is 0.1 % TFA in acetonitrile. A fraction at time 16.642 min containing the expected peptide, as judged by mass spectrometry, was pooled and lyophilized. The peptide was then subjected to proteasome digestion and mass spectrum analysis essentially as described above. Prominent peaks from the mass spectra are summarized in Table 1.

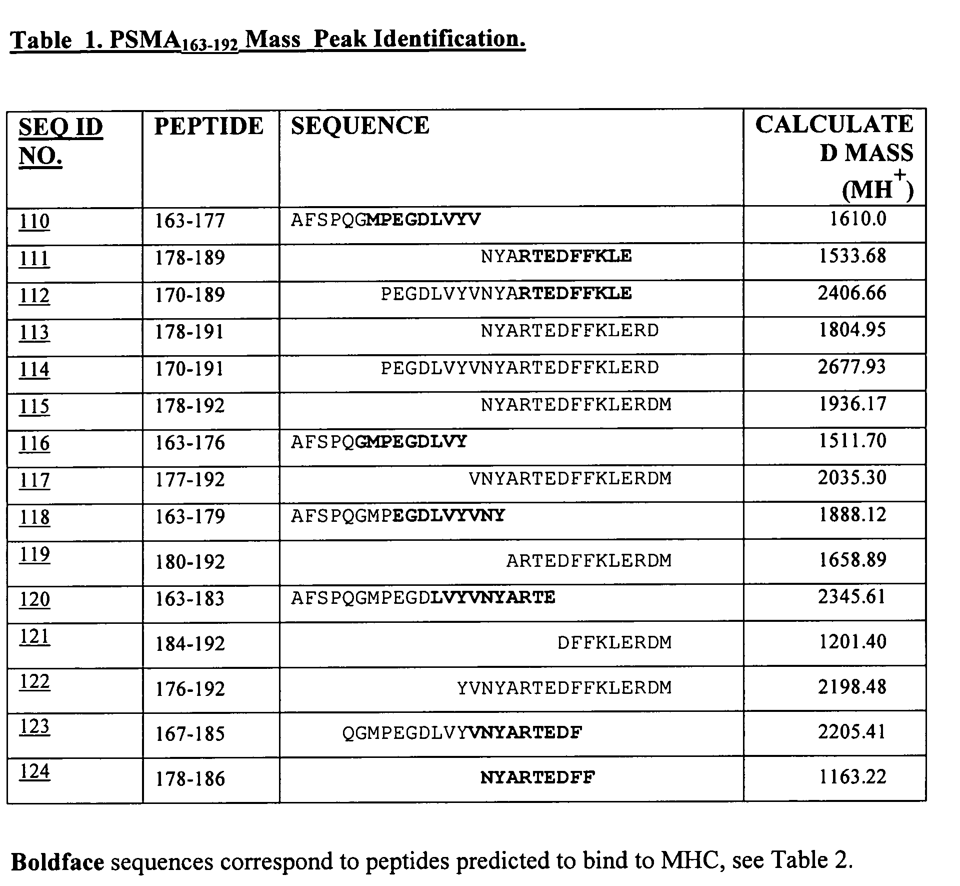

### N-terminal Pool Sequence Analysis

One aliquot at one hour of the proteasomal digestion was subjected to N-terminal amino acid sequence analysis by an ABI 473A Protein Sequencer (Applied Biosystems, Foster City, CA). Determination of the sites and efficiencies of cleavage was based on consideration of the sequence cycle, the repetitive yield of the protein sequencer, and the relative yields of amino acids unique in the analyzed sequence. That is if the unique (in the analyzed sequence) residue X appears only in the nth cycle a cleavage site exists n-1 residues before it in the N-terminal direction. In addition to helping resolve any ambiguity in the assignment of mass to sequences, these data also provide a more reliable indication of the relative yield of the various fragments than does mass spectrometry.

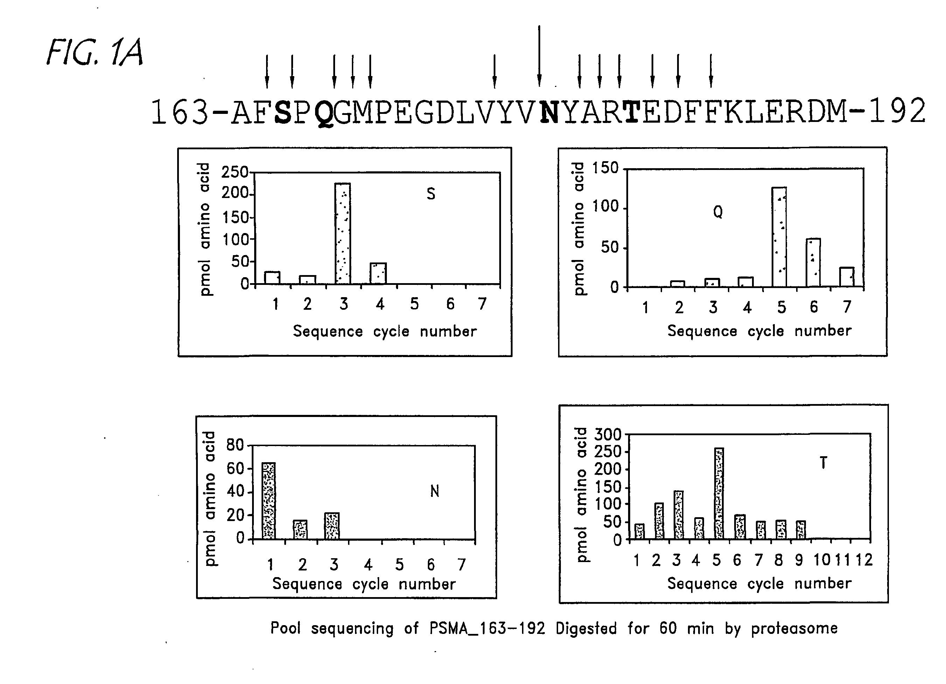

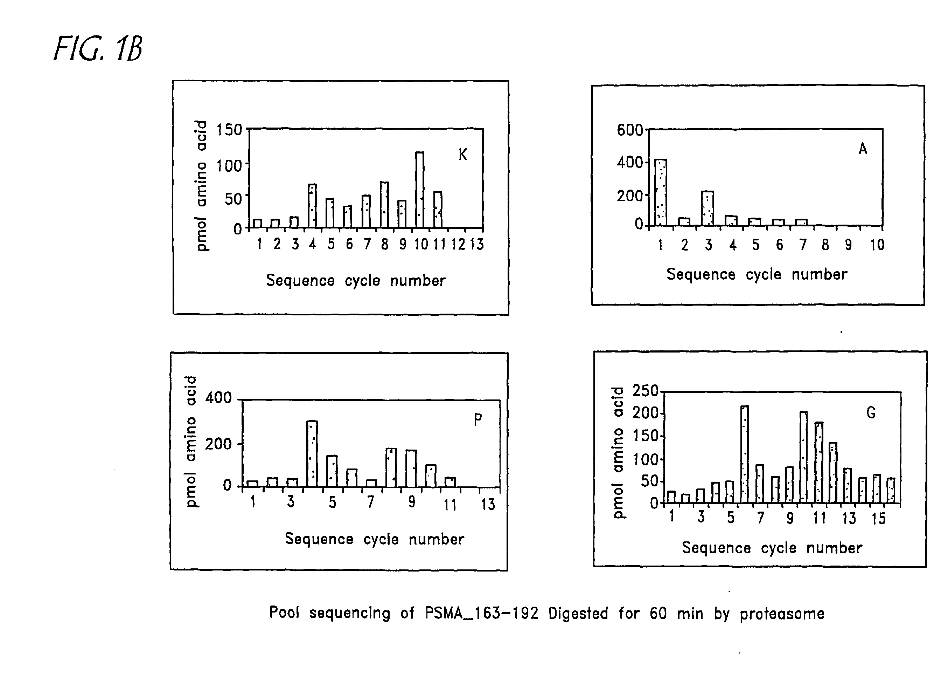

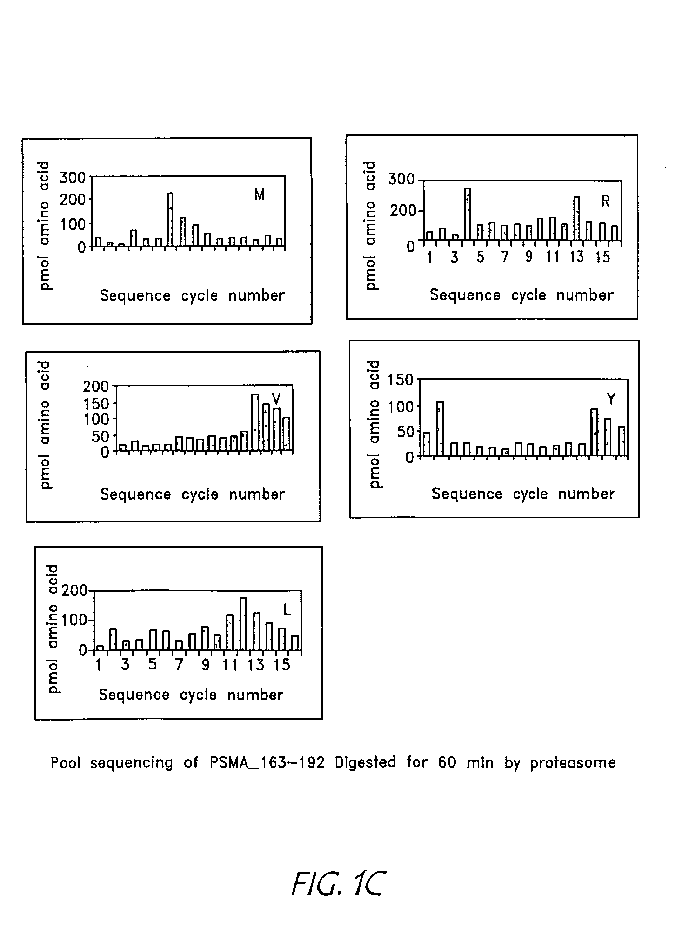

For PSMA₁₆₃₋₁₉₂ (SEQ ID NO. 3) this pool sequencing supports a single major cleavage site after V₁₇₇ and several minor cleavage sites, particularly one after Y₁₇₉. Reviewing the results presented in figures 1A-C reveals the following:
S at the 3^{rd} cycle indicating presence of the N-terminus of the substrate.
Q at the 5^{th} cycle indicating presence of the N-terminus of the substrate.
N at the 1^{st} cycle indicating cleavage after V₁₇₇.
N at the 3^{rd} cycle indicating cleavage after V₁₇₅. Note the fragment 176-192 in Table 1.
T at the 5^{th} cycle indicating cleavage after V₁₇₇.
T at the 1^{st} -3^{rd} cycles, indicating increasingly common cleavages after R₁₈₁, A₁₈₀ and Y₁₇₉. Only the last of these correspond to peaks detected by mass spectrometry; 163-179 and 180-192, see Table 1. The absence of the others can indicate that they are on fragments smaller than were examined in the mass spectrum.
K at the 4^{th}, 8^{th}, and 10^{th} cycles indicating cleavages after E₁₈₃, Y₁₇₉, and V₁₇₇, respectively, all of which correspond to fragments observed by mass spectroscopy. See Table 1.
A at the 1^{st} and 3^{rd} cycles indicating presence of the N-terminus of the substrate and cleavage after V₁₇₇, respectively.
P at the 4^{th} and 8^{th} cycles indicating presence of the N-terminus of the substrate.
G at the 6^{th} and 10^{th} cycles indicating presence of the N-terminus of the substrate.
M at the 7^{th} cycle indicating presence of the N-terminus of the substrate and/or cleavage after F₁₈₅.
M at the 15^{th} cycle indicating cleavage after V₁₇₇.
The 1^{st} cycle can indicate cleavage after D₁₉₁, see Table 1.
R at the 4^{th} and 13^{th} cycle indicating cleavage after V₁₇₇.
R at the 2^{nd} and 11^{th} cycle indicating cleavage after Y₁₇₉.
V at the 2^{nd}, 6^{th}, and 13^{th} cycle indicating cleavage after V₁₇₅, M₁₆₉ and presence of the N-terminus of the substrate, respectively. Note fragments beginning at 176 and 170 in Table 1.
Y at the 1^{st}, 2^{nd}, and 14^{th} cycles indicating cleavage after V₁₇₅, V₁₇₇, and presence of the N-terminus of the substrate, respectively.
L at the11^{th} and 12^{th} cycles indicating cleavage after V₁₇₇, and presence of the N-terminus of the substrate, respectively, is the interpretation most consistent with the other data. Comparing to the mass spectrometry results we see that L at the 2^{nd}, 5^{th}, and 9^{th} cycles is consistent with cleavage after F₁₈₆, E₁₈₃ or M₁₆₉, and Y₁₇₉, respectively. See Table 1.

### Epitope Identification

Fragments co-C-terminal with 8-10 amino acid long sequences predicted to bind HLA by the SYFPEITHI or NIH algorithms were chosen for further analysis. The digestion and prediction steps of the procedure can be usefully practiced in any order. Although the substrate peptide used in proteasomal digest described here was specifically designed to include a predicted HLA-A1 binding sequence, the actual products of digestion can be checked after the fact for actual or predicted binding to other MHC molecules. Selected results are shown in Table 2.

**Table 2.**

| **Predicted HLA binding by proteasomally generated fragments** | | | | |
|---|---|---|---|---|
| **SEQ ID NO** | **PEPTIDE** | **HLA** | **SYFPEITHI** | **NIH** |
| 5 & (6) | (G)MPEGDLV YV | A*0201 | 17 (27) | (2605) |
| | | B*0702 | 20 | <5 |
| | | B*5101 | 22 | 314 |
| 7 & (8) 9 | (Q)GMPEGDL VY MPEGDLVY | A1 | 24 (26) | <5 |
| | | A3 | 16 (18) | 36 |
| | | B*2705 | 17 | 25 |
| | | B*5101 | 15 | NP† |
| 10 & (11) | (P)EGDLVYV NY | A1 | 27 (15) | 12 |
| | | A26 | 23 (17) | NP |
| 12 | LVYVNYART E | A3 | 21 | <5 |
| 13 & (14) | (Y)VNYARTE DF | A26 | (20) | NP |
| | | B*08 | 15 | <5 |
| | | B*2705 | 12 | 50 |
| 15 16 | NYARTEDFF YARTEDFF | A24 | NP† | 100 |
| | | Cw*0401 | NP | 120 |
| | | B*08 | 16 | <5 |
| 17 | RTEDFFKLE | A1 | 21 | <5 |
| | | A26 | 15 | NP |

| | | | | |
|---|---|---|---|---|
| †No prediction | | | | |

### HLA-A*0201 binding assay:

Binding of the candidate epitope PSMA₁₆₈₋₁₇₇, GMPEGDLVYV, (SEQ ID NO. 6) to HLA-A2.1 was assayed using a modification of the method of Stauss et al., (Proc Natl Acad Sci USA 89(17):7871-5 (1992)). Specifically, T2 cells, which express empty or unstable MHC molecules on their surface, were washed twice with Iscove's modified Dulbecco's medium (IMDM) and cultured overnight in serum-free AIM-V medium (Life Technologies, Inc., Rockville, MD) supplemented with human β2-microglobulin at 3µg/ml (Sigma, St. Louis, MO) and added peptide, at 800, 400, 200, 100, 50, 25, 12.5, and 6.25 µg/ml.in a 96-well flat-bottom plate at 3x10⁵ cells/200 µl/well. Peptide was mixed with the cells by repipeting before distributing to the plate (alternatively peptide can be added to individual wells), and the plate was rocked gently for 2 minutes. Incubation was in a 5% CO₂ incubator at 37°C. The next day the unbound peptide was removed by washing twice with serum free RPMI medium and a saturating amount of anti-class I HLA monoclonal antibody, fluorescein isothiocyanate (FITC)-conjugated anti-HLA A2, A28 (One Lambda, Canoga Park, CA) was added. After incubation for 30 minutes at 4°C, cells were washed 3 times with PBS supplemented with 0.5% BSA, 0.05%(w/v) sodium azide, pH 7.4-7.6 (staining buffer). (Alternatively W6/32 (Sigma) can be used as the anti-class I HLA monoclonal antibody the cells washed with staining buffer and then incubated with fluorescein isothiocyanate (FITC)-conjugated goat F(ab') antimouse-IgG (Sigma) for 30 min at 4°C and washed 3 times as before.) The cells were resuspended in 0.5 ml staining buffer. The analysis of surface HLA-A2.1 molecules stabilized by peptide binding was performed by flow cytometry using a FACScan (Becton Dickinson, San Jose, CA). If flow cytometry is not to be performed immediately the cells can be fixed by adding a quarter volume of 2% paraformaldehyde and storing in the dark at 4°C.

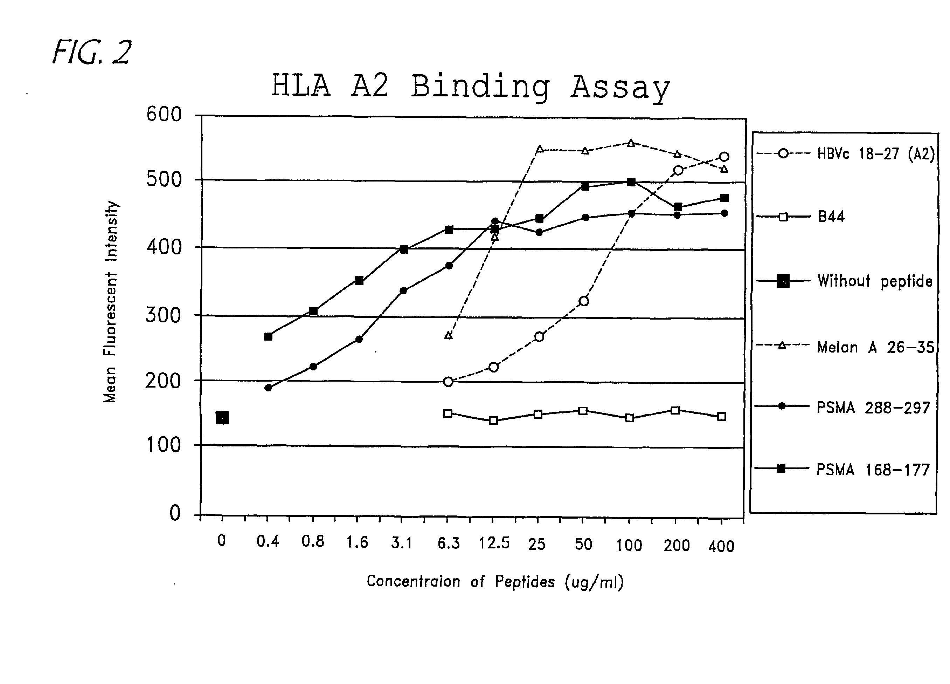

As seen in figure 2, this epitope exhibits significant binding at even lower concentrations than the positive control peptides. The Melan-A peptide used as a control in this assay (and throughout this disclosure), ELAGIGILTV (SEQ ID NO: 106), is actually a variant of the natural sequence (EAAGIGILTV; SEQ ID NO: 107)) and exhibits a high affinity in this assay. The known A2.1 binder FLPSDYFPSV (HBV₁₈₋₂₇; SEQ ID NO: 107) was also used as a positive control. An HLA-B44 binding peptide, AEMGKYSFY (SEQ ID NO: 109), was used as a negative control. The fluorescence obtained from the negative control was similar to the signal obtained when no peptide was used in the assay. Positive and negative control peptides were chosen from Table 18.3.1 in *Current Protocols in Immunology* p. 18.3.2, John Wiley and Sons, New York, 1998.

### Example 1.2

### Cluster Analysis (PSMA₂₈₁₋₃₁₀).

Another peptide, RGIAEAVGLPSIPVHPIGYYDAQKLLEKMG, PSMA₂₈₁₋₃₁₀, (SEQ ID NO. 18), containing an A1 epitope cluster from prostate specific membrane antigen, PSMA₂₈₃₋₃₀₇ (SEQ ID NO. 19), was synthesized using standard solid-phase F-moc chemistry on a 433A ABI Peptide synthesizer. After side chain deprotection and cleavage from the resin, peptide in ddH2O was run on a reverse-phase preparative HPLC C 18 column at following conditions: linear AB gradient (5% B/min) at a flow rate of 4 ml/min, where eluent A is 0.1% aqueous TFA and eluent B is 0.1 % TFA in acetonitrile. A fraction at time 17.061 min containing the expected peptide as judged by mass spectrometry, was pooled and lyophilized. The peptide was then subjected to proteasome digestion and mass spectrum analysis essentially as described above. Prominent peaks from the mass spectra are summarized in Table 3.

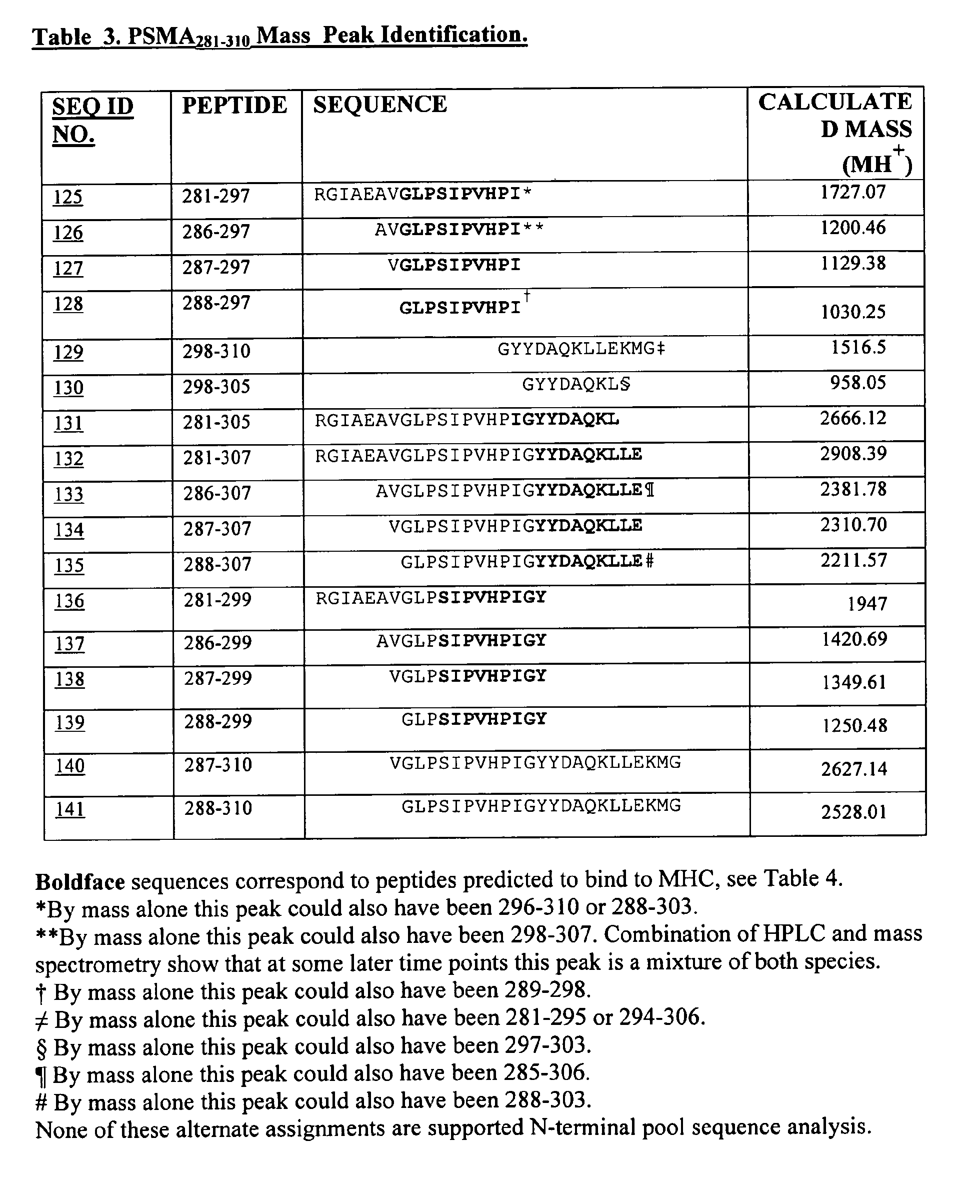

### N-terminal Pool Sequence Analysis

One aliquot at one hour of the proteasomal digestion (see Example 3 part 3 above) was subjected to N-terminal amino acid sequence analysis by an ABI 473A Protein Sequencer (Applied Biosystems, Foster City, CA). Determination of the sites and efficiencies of cleavage was based on consideration of the sequence cycle, the repetitive yield of the protein sequencer, and the relative yields of amino acids unique in the analyzed sequence. That is if the unique (in the analyzed sequence) residue X appears only in the nth cycle a cleavage site exists n-1 residues before it in the N-terminal direction. In addition to helping resolve any ambiguity in the assignment of mass to sequences, these data also provide a more reliable indication of the relative yield of the various fragments than does mass spectrometry.

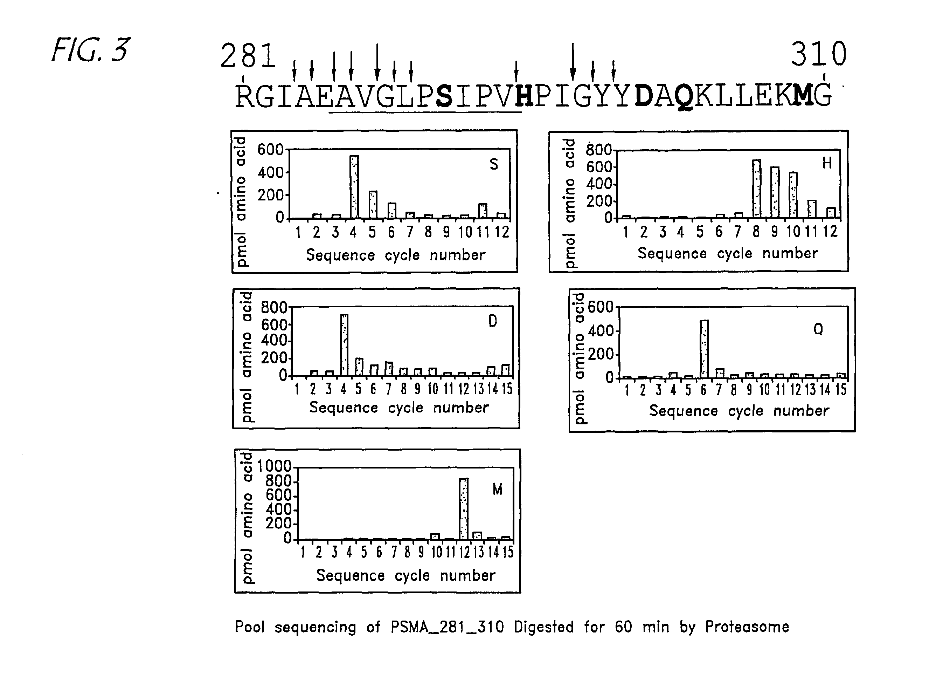

For PSMA₂₈₁₋₃₁₀ (SEQ ID NO. 18) this pool sequencing supports two major cleavage sites after V₂₈₇ and I₂₉₇ among other minor cleavage sites. Reviewing the results presented in Fig. 3 reveals the following:
S at the 4^{th} and 11^{th} cycles indicating cleavage after V₂₈₇ and presence of the N-terminus of the substrate, respectively.
H at the 8^{th} cycle indicating cleavage after V₂₈₇. The lack of decay in peak height at positions 9 and 10 versus the drop in height present going from 10 to 11 can suggest cleavage after A₂₈₆ and E₂₈₅ as well, rather than the peaks representing latency in the sequencing reaction.
D at the 2^{nd}, 4^{th}, and 7^{th} cycles indicating cleavages after Y₂₉₉, I₂₉₇, and V₂₉₄, respectively. This last cleavage is not observed in any of the fragments in Table 4 or in the alternate assignments in the notes below.
Q at the 6^{th} cycle indicating cleavage after I₂₉₇.
M at the 10^{th} and 12^{th} cycle indicating cleavages after Y₂₉₉ and I₂₉₇, respectively.

### Epitope Identification

Fragments co-C-terminal with 8-10 amino acid long sequences predicted to bind HLA by the SYFPEITHI or NIH algorithms were chosen for further study. The digestion and prediction steps of the procedure can be usefully practiced in any order. Although the substrate peptide used in proteasomal digest described here was specifically designed to include a predicted HLA-A1 binding sequence, the actual products of digestion can be checked after the fact for actual or predicted binding to other MHC molecules. Selected results are shown in Table 4.

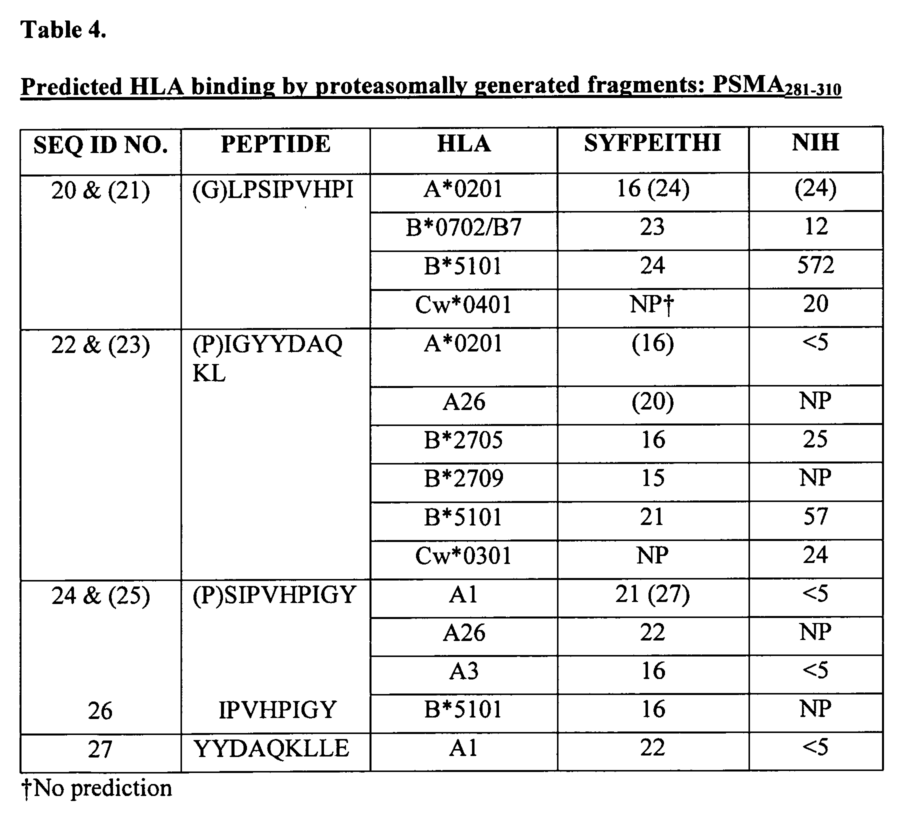

As seen in Table 4, N-terminal addition of authentic sequence to epitopes can often generate still useful, even better epitopes, for the same or different MHC restriction elements. Note for example the pairing of (G)LPSIPVHPI with HLA-A*0201, where the 10-mer can be used as a vaccine useful with several MHC types by relying on N-terminal trimming to create the epitopes for HLA-B7, -B*5101, and Cw*0401.

### HLA-A*0201 binding assay:

HLA-A*0201 binding studies were preformed with PSMA₂₈₈₋₂₉₇, GLPSIPVHPI, (SEQ ID NO. 21) essentially as described in Example 1.1 above. As seen in figure 2, this epitope exhibits significant binding at even lower concentrations than the positive control peptides.

### Example 1.3

### Cluster Analysis (PSMA₄₅₄₋₄₈₁).

Another peptide, SSIEGNYTLRVDCTPLMYSLVHLTKEL, PSMA₄₅₄₋₄₈₁, (SEQ ID NO. 28) containing an epitope cluster from prostate specific membrane antigen, was synthesized by MPS (purity >95%) and subjected to proteasome digestion and mass spectrum analysis as described above. Prominent peaks from the mass spectra are summarized in Table 5.

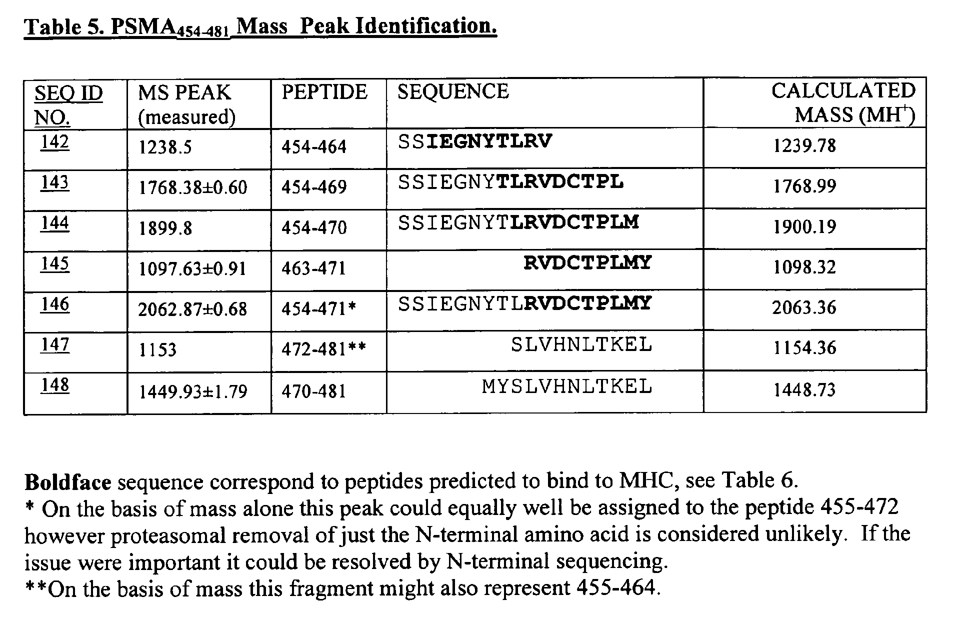

### Epitope Identification

Fragments co-C-terminal with 8-10 amino acid long sequences predicted to bind HLA by the SYFPEITHI or NIH algorithms were chosen for further study. The digestion and prediction steps of the procedure can be usefully practiced in any order. Although the substrate peptide used in proteasomal digest described here was specifically designed to include predicted HLA-A2.1 binding sequences, the actual products of digestion can be checked after the fact for actual or predicted binding to other MHC molecules. Selected results are shown in Table 6.

**Table 6.**

| **Predicted HLA binding by proteasomally generated fragments** | | | | |
|---|---|---|---|---|
| **SEQ ID NO** | **PEPTIDE** | **HLA** | **SYFPEITHI** | **NIH** |
| 29 & (30) | (S)IEGNYTLRV | A1 | (19) | <5 |
| 31 | EGNYTLRV | A*0201 | 16 (22) | <5 |
| | | B*5101 | 15 | NP† |
| 32 & (33) | (Y)TLRVDCTPL | A*0201 | 20 (18) | (5) |
| | | A26 | 16(18) | NP |
| | | B7 | 14 | 40 |
| | | B8 | 23 | <5 |
| | | B*2705 | 12 | 30 |
| | | Cw*0301 | NP | (30) |
| 34 | LRVDCTPLM | B*2705 | 20 | 600 |
| | | B*2709 | 20 | NP |
| 35 & (36) | (L)RVDCTPLMY | A1 | 32 (22) | 125 (13.5) |
| | | A3 | 25 | <5 |
| | | A26 | 22 | NP |
| | | B*2702 | NP | (200) |
| | | B*2705 | 13 (NP) | (1000) |

| | | | | |
|---|---|---|---|---|
| †No prediction | | | | |

As seen in Table 6, N-terminal addition of authentic sequence to epitopes can often generate still useful, even better epitopes, for the same or different MHC restriction elements. Note for example the pairing of (L)RVDCTPLMY (SEQ ID NOS 35 and (36)) with HLA-B*2702/5, where the 10-mer has substantial predicted halftimes of dissociation and the co-C-terminal 9-mer does not. Also note the case of SIEGNYTLRV (SEQ ID NO 30) a predicted HLA-A*0201 epitope which can be used as a vaccine useful with HLA-B*5101 by relying on N-terminal trimming to create the epitope.

### HLA-A*0201 binding assay

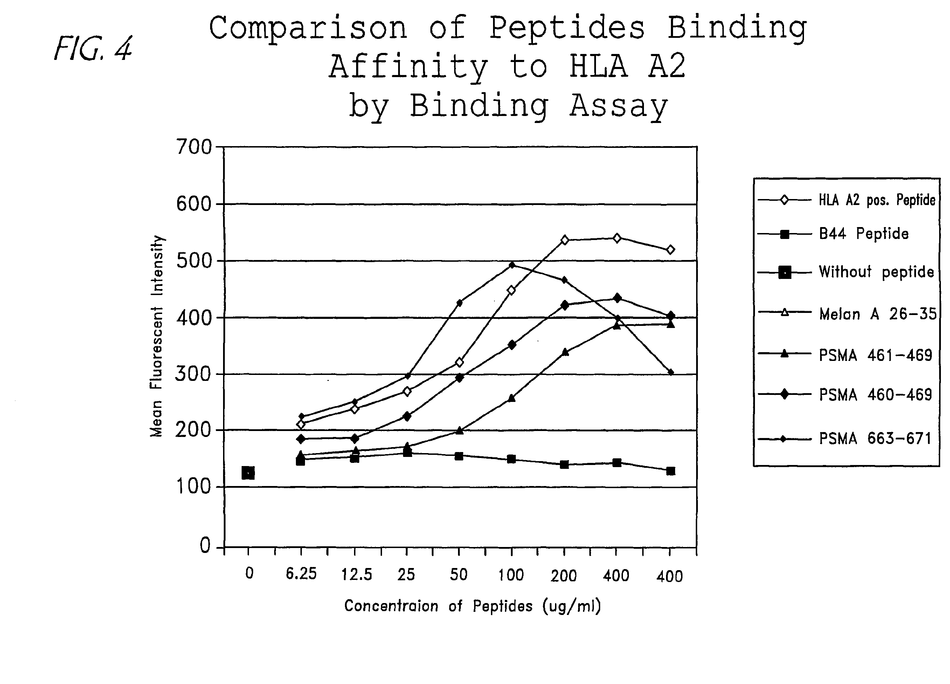

HLA-A*0201 binding studies were preformed, essentially as described in Example 1.1 above, with PSMA₄₆₀₋₄₆₉, YTLRVDCTPL, (SEQ ID NO.33). As seen in figure 4, this epitope was found to bind HLA-A2.1 to a similar extent as the known A2.1 binder FLPSDYFPSV (HBV₁₈₋₂₇; SEQ ID NO: 108) used as a positive control. Additionally, PSMA₄₆₁₋₄₆₉, (SEQ ID NO. 32) binds nearly as well.

### ELISPOT analysis: PSMA₄₆₃₋₄₇₁ (SEQ ID NO. 35)

The wells of a nitrocellulose-backed microtiter plate were coated with capture antibody by incubating overnight at 4°C using 50 µl/well of 4µg/ml murine anti-human □-IFN monoclonal antibody in coating buffer (35 mM sodium bicarbonate, 15 mM sodium carbonate, pH 9.5). Unbound antibody was removed by washing 4 times 5 min. with PBS. Unbound sites on the membrane then were blocked by adding 200µl/well of RPMI medium with 10% serum and incubating 1 hr. at room temperature. Antigen stimulated CD8⁺ T cells, in 1:3 serial dilutions, were seeded into the wells of the microtiter plate using 100µl/well, starting at 2x10⁵ cells/well. (Prior antigen stimulation was essentially as described in Scheibenbogen, C. et al. *Int. J. Cancer* 71:932-936, 1997; which is incorporated herein by reference in its entirety.) PSMA₄₆₂₋₄₇₁ (SEQ ID NO. 36) was added to a final concentration of 10µg/ml and IL-2 to 100 U/ml and the cells cultured at 37°C in a 5% CO₂, water-saturated atmosphere for 40 hrs. Following this incubation the plates were washed with 6 times 200 µl/well of PBS containing 0.05% Tween-20 (PBS-Tween). Detection antibody, 50µl/well of 2g/ml biotinylated murine anti-human □-IFN monoclonal antibody in PBS+10% fetal calf serum, was added and the plate incubated at room temperature for 2 hrs. Unbound detection antibody was removed by washing with 4 times 200 µl of PBS-Tween. 100µl of avidin-conjugated horseradish peroxidase (Pharmingen, San Diego, CA) was added to each well and incubated at room temperature for 1 hr. Unbound enzyme was removed by washing with 6 times 200 µl of PBS-Tween. Substrate was prepared by dissolving a 20 mg tablet of 3-amino 9-ethylcoarbasole in 2.5 ml ofN, N-dimethylformamide and adding that solution to 47,5 ml of 0.05 M phosphate-citrate buffer (pH 5.0). 25 µl of 30% H₂O₂ was added to the substrate solution immediately before distributing substrate at100 µl/well and incubating the plate at room temperature. After color development (generally 15-30 min.), the reaction was stopped by washing the plate with water. The plate was air dried and the spots counted using a stereomicroscope.

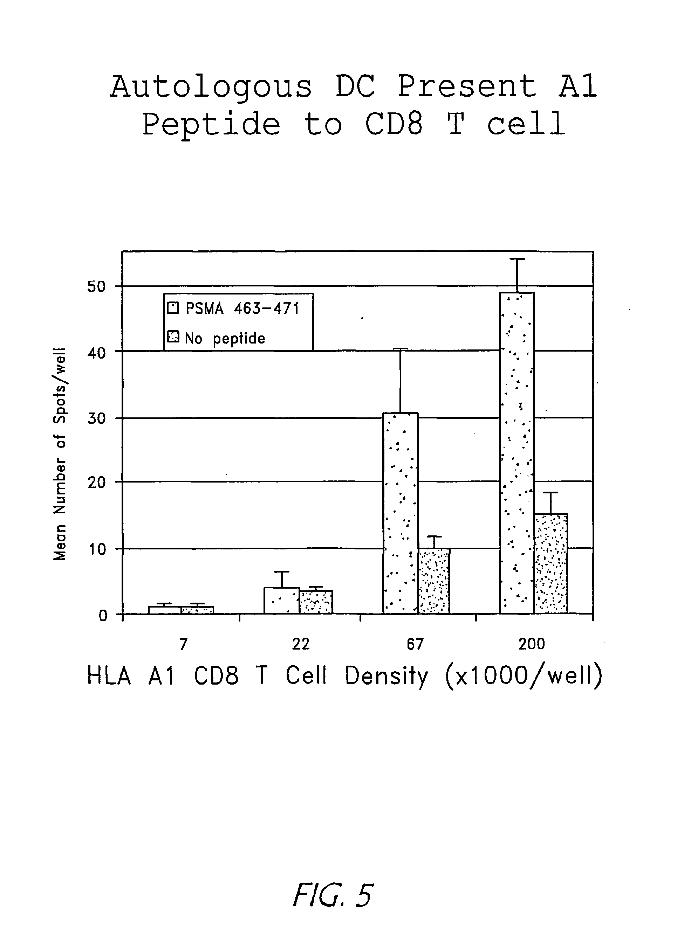

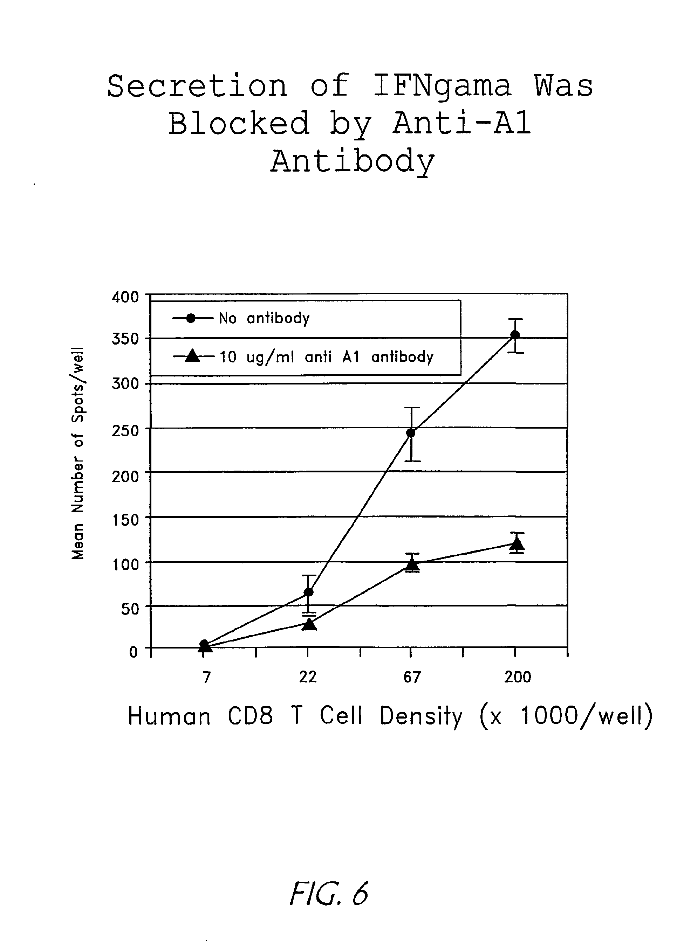

Figure 5 shows the detection of PSMA₄₆₃₋₄₇₁ (SEQ ID NO. 35)-reactive HLA-A1⁺ CD8⁺ T cells previously generated in cultures of HLA-A1⁺ CD8⁺ T cells with autologous dendritic cells plus the peptide. No reactivity is detected from cultures without peptide (data not shown). In this case it can be seen that the peptide reactive T cells are present in the culture at a frequency between 1 in 2.2x10⁴ and 1 in 6.7x10⁴. That this is truly an HLA-A1-restricted response is demonstrated by the ability of anti-HLA-A1 monoclonal antibody to block □-IFN production; see figure 6.

### Example 1.4

### Cluster Analysis (PSMA₆₅₃₋₆₈₇).

Another peptide, FDKSNPIVLRMMNDQLMFLERAFIDPLGLPDRPFY PSMA₆₅₃₋₆₈₇, (SEQ ID NO. 37) containing an A2 epitope cluster from prostate specific membrane antigen, PSMA₆₆₀₋₆₈₁ (SEQ ID NO. 38), was synthesized by MPS (purity >95%) and subjected to proteasome digestion and mass spectrum analysis as described above. Prominent peaks from the mass spectra are summarized in Table 7.

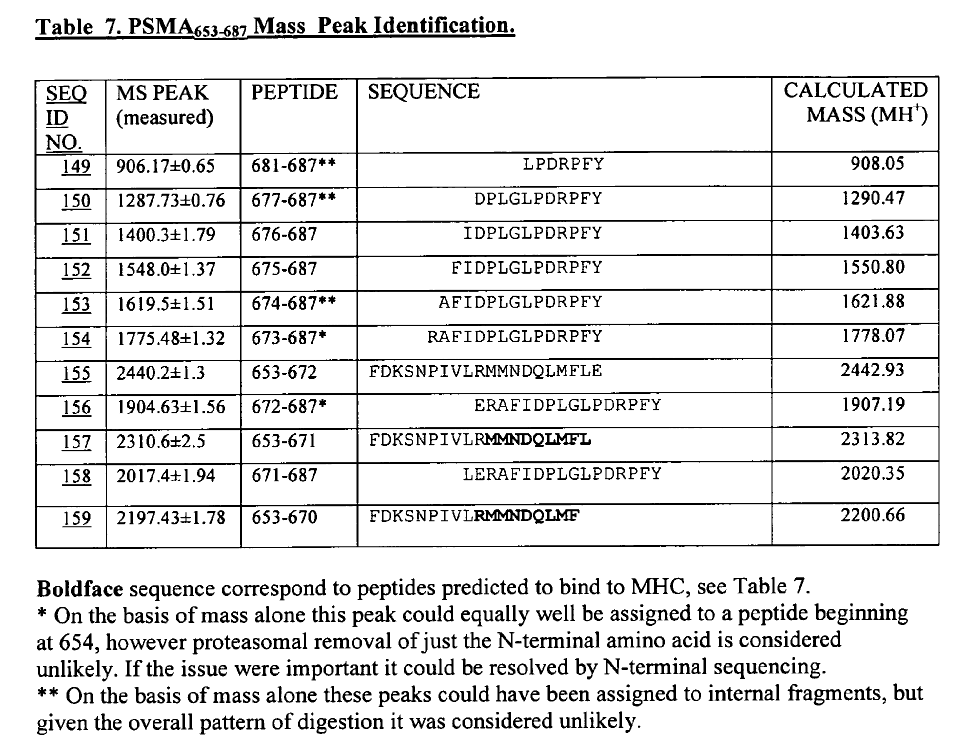

### Epitope Identification

Fragments co-C-terminal with 8-10 amino acid long sequences predicted to bind HLA by the SYFPEITHI or NIH algorithms were chosen for further study. The digestion and prediction steps of the procedure can be usefully practiced in any order. Although the substrate peptide used in proteasomal digest described here was specifically designed to include predicted HLA-A2.1 binding sequences, the actual products of digestion can be checked after the fact for actual or predicted binding to other MHC molecules. Selected results are shown in Table 8.

**Table 8.**

| **Predicted HLA binding by proteasomally generated fragments** | | | | |
|---|---|---|---|---|
| **SEQ ID NO** | **PEPTIDE** | **HLA** | **SYFPEITHI** | **NIH** |
| 39 & (40) | (R)MMNDQLM FL | A*0201 | 24 (23) | 1360 (722) |
| | | A*0205 | NP† | 71 (42) |
| | | A26 | 15 | NP |
| | | B*2705 | 12 | 50 |
| 41 | RMMNDQLMF | B*2705 | 17 | 75 |

| | | | | |
|---|---|---|---|---|
| †No prediction | | | | |

As seen in Table 8, N-terminal addition of authentic sequence to epitopes can generate still useful, even better epitopes, for the same or different MHC restriction elements. Note for example the pairing of (R)MMNDQLMFL (SEQ ID NOS. 39 and (40)) with HLA-A*02, where the 10-mer retains substantial predicted binding potential.

### HLA-A*0201 binding assay

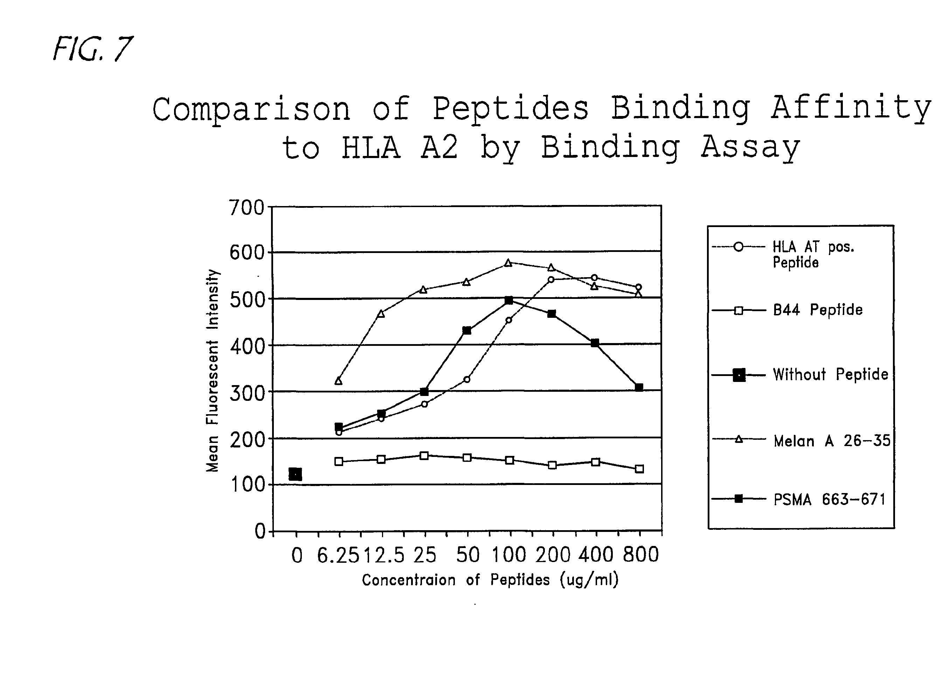

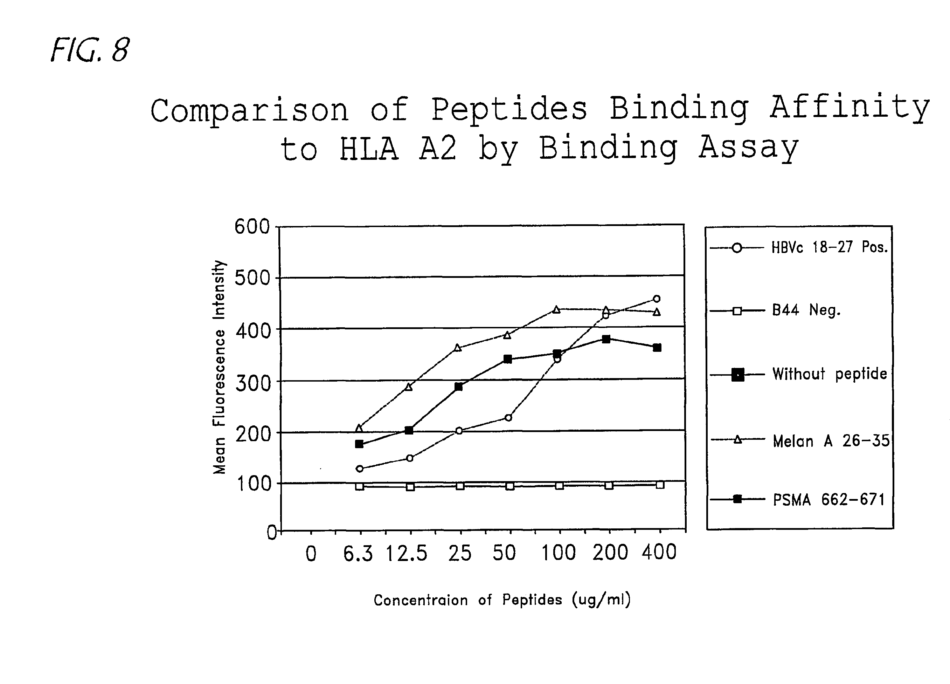

HLA-A*0201 binding studies were preformed, essentially as described in Example 1.1 above, with PSMA₆₆₃₋₆₇₁, (SEQ ID NO. 39) and PSMA₆₆₂₋₆₇₁, RMMNDQLMFL (SEQ NO. 67). As seen in figures 4, 7 and 8, this epitope exhibits significant binding at even lower concentrations than the positive control peptide (FLPSDYFPSV (HBV₁₈₋₂₇); SEQ ID NO. 108). Though not run in parallel, comparison to the controls suggests that PSMA₆₆₂₋₆₇₁ (which approaches the MelanA peptide in affinity) has the superior binding activity of these two PSMA peptides.

### Example 2.

A multi-center clinical study is carried out using compositions as disclosed herein. The results of the study show the compositions to be useful and effective for debulking solid tumors and for generally inducing anti-angiogenic activity.

### Example 3 Evaluation of a PSMA composition in the Xenotransplanted Human Vasculature Model

### Generation of target antigen-reactive CTL

### A. In vivo immunization of mice.

HHD1 transgenic A*0201 mice (Pascolo, S., et al. *J. Exp. Med.* 185:2043-2051, 1997) were anesthetized and injected subcutaneously at the base of the tail, avoiding lateral tail veins, using 100 µl containing 100 nmol of PSMA₂₈₈₋₂₉₇ (SEQ ID NO. 21) and 20 µg of a HTL epitope peptide in PBS emulsified with 50 µl of IFA (incomplete Freund's adjuvant).

### B. Preparation of stimulating cells (LPS blasts).

Using spleens from 2 naive mice for each group of immunized mice, un-immunized mice were sacrificed and their carcasses placed in alcohol. Using sterile instruments, the top dermal layer of skin on the mouse's left side (lower mid-section) was cut through, exposing the peritoneum. The peritoneum was saturated with alcohol, and the spleen aseptically extracted. The spleens were placed in a petri dish with serum-free media. Splenocytes were isolated by using sterile plungers from 3 ml syringes to mash the spleens. Cells were collected in a 50 ml conical tubes in serum-free media, rinsing dish well. Cells were centrifuged (12000 rpm, 7 min) and washed one time with RPMI. Fresh spleen cells were resuspended to a concentration of 1x10⁶ cells per ml in RPMI-10%FCS (fetal calf serum). 25g/ml lipopolysaccharide and 7 µg/ml Dextran Sulfate were added. Cell were incubated for 3 days in T-75 flasks at 37°C, with 5% CO₂. Splenic blasts were collected in 50 ml tubes pelleted (12,000 rpm, 7 min) and resuspended to 3X10^{7/}ml in RPMI. The blasts were pulsed with the priming peptide at 50 µg/ml, RT 4hr. mitomycin C-treated at 25µg/ml, 37°C, 20 min and washed three times with DMEM.

### C. In vitro stimulation.

Three days after LPS stimulation of the blast cells and the same day as peptide loading, the primed mice were sacrificed (at 14 days post immunization) to remove spleens as above. 3x10⁶ splenocytes were co-cultured with 1x10⁶ LPS blasts/well in 24-well plates at 37°C, with 5% CO₂ in DMEM media supplemented with 10% FCS, 5x10⁻⁵ M β (beta)-mercaptoethanol, 100µg/ml streptomycin and 100 IU/ml penicillin. Cultures were fed 5% (vol/vol) ConA supernatant on day 3 and can be transferred on day 7. An aliquot of the CTL are also tested in a standard chromium release assay to ensure activity.

### Implantation and Adoptive transfer

1x10⁶ telomerase-transformed HDMEC in 10 µl of EGM-2-VM medium (Clonetics, San Diego, CA) are mixed with 0.5 ml of MATRIGEL (Becton Dickinson) on ice. The mixture is injected subcutaneously, through a 25 gauge needle, along the ventral midline of the thorax of SCID mice. One week later 1x10⁷ T cells (target epitope-reactive or sham-immunized) in 0.2 ml are injected intravenously (alternatively they can be injected intraperitoneally).

### Assessment (Micromorphometry)

At one and two weeks after transfer remove implants, fix in 10% buffered overnight, embed in paraffin, and section. For immunofluorescence detection of human microvessels using anti-human type IV collagen IgG and fluorescently-labeled secondary antibody, deparifinize and retrieve antigen by microwaving thin sections 2x7 minutes in 10mM citric acid, pH 6.0. Vessel density is assessed as a function of the average number of positively stained annular structures observed in five separate, randomly selected 20x fields-of-view, from at least three sections per implant.

### Example 4. A Fibronectin ED-B Vaccine in the HLA-transgenic Mouse Model

### A. Establishment of tumor

M1 tumor cells grown in complete RPMI plus 10% serum were harvested and washed with PBS by centrifugation. The cells were suspended in PBS at 5x10⁶ cells/ml and 0.5 ml of the suspension (early passage) was injected subcutaneously into the abdomen.

### B. Vaccination

A nucleotide sequence encoding an HLA-A2-restricted fibronectin ED-B domain-derived housekeeping epitope, for example ED-B₂₉₋₃₈ (SEQ ID NO. 103), is inserted into an appropriate vaccine vector (e.g. pVAX1 (Invitrogen Inc, Carlsbad, CA) or one of the vectors described in U.S. Patent Application No. 09/561,572 entitled "EXPRESSION VECTORS ENCODING EPITOPES OF TARGET-ASSOCIATED ANTIGENS," filed on April 28, 2001. HHD-A2 mice are injected intranodally in the inguinal lymph node with 0, 2, 10, 50, 100, and 200 µg of vector in PBS every other day over 8 days (4 injections) alternating sides for each injection (single dosage per mouse or group of mice). Injection series are started the day of tumor cell injection, at 2 weeks before, and at 4 and 10 weeks after.

### C. Evaluation

At approximately 12 weeks after injection of tumor cells visible tumors are observed in the mice receiving the vehicle instead of the vaccine. Effectiveness of the vaccine is expressed as the proportion of vaccinated animals that fail to develop a tumor in the same time frame, the relative size of tumors at the same time point, the delay in time before tumors appear in the vaccinated animals, and the dose and number of composition cycles needed to inhibit or prevent the establishment of tumor.

### D. Alternative schedule

The availability of more aggressive later passage M1 cells enables a more compressed experimental schedule. Instead mice are vaccinated on the day of tumor cell inoculation, 1 and 2 weeks before, and 3 or 4 days after injections of 1x10⁶ cells. Effectiveness of vaccination is assessed at approximately 10 days after tumor cell inoculation.

### E. Immunization with peptide

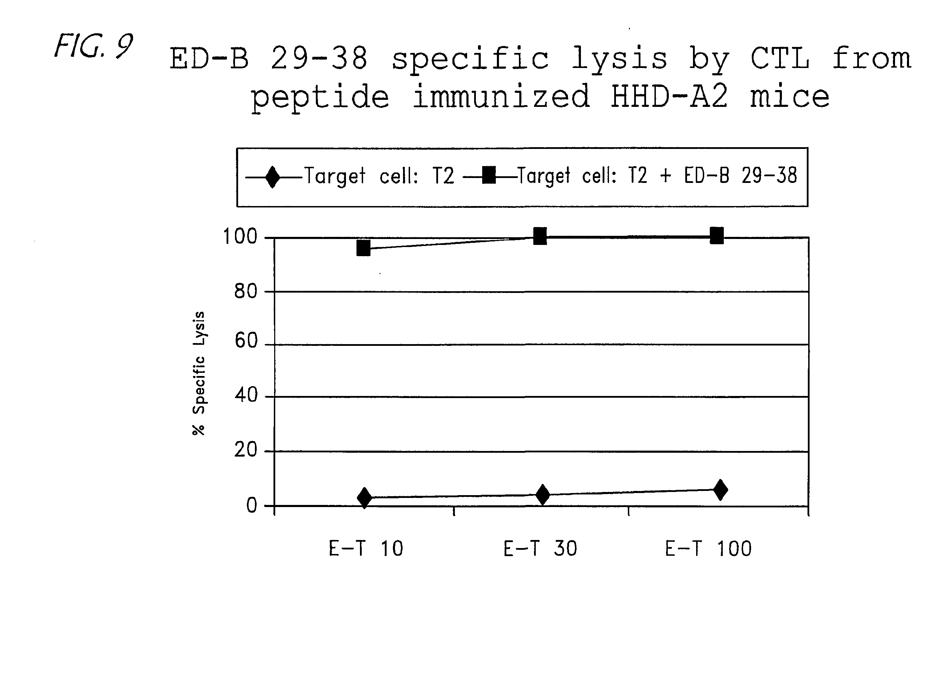

HHD-A2 mice were immunized with ED-B29-38 (SEQ ID NO. 103) in complete Freund's adjuvants and spleen cells were harvested and re-stimulated *in vitro* using standard methodology. The resulting CTL were able to specifically lyse peptide pulsed T2 cells, which are HLA-A2+ (Fig. 9).

### Example 5. Epitopes and Epitope Clusters

Table 9 discloses epitopes and epitope clusters from PSMA and ED-B that can be useful in construction of compositions according to the present invention.

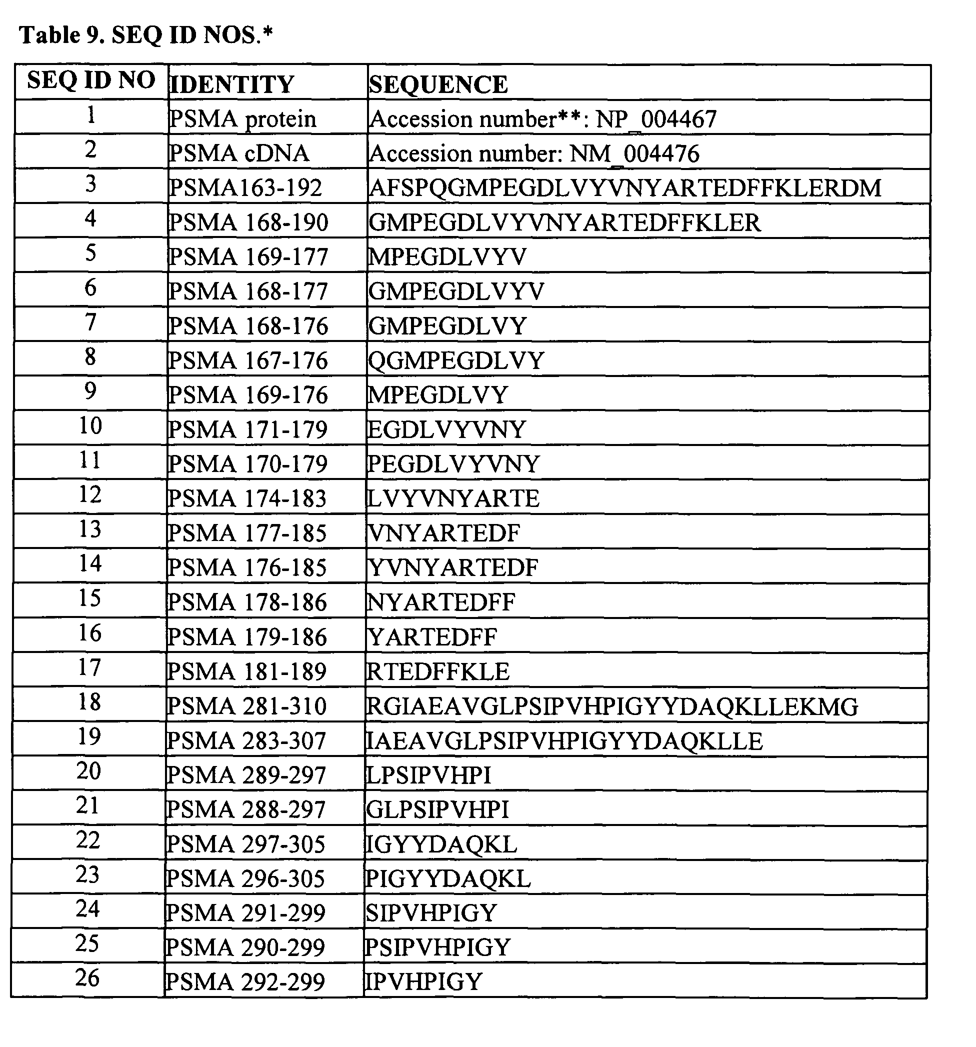

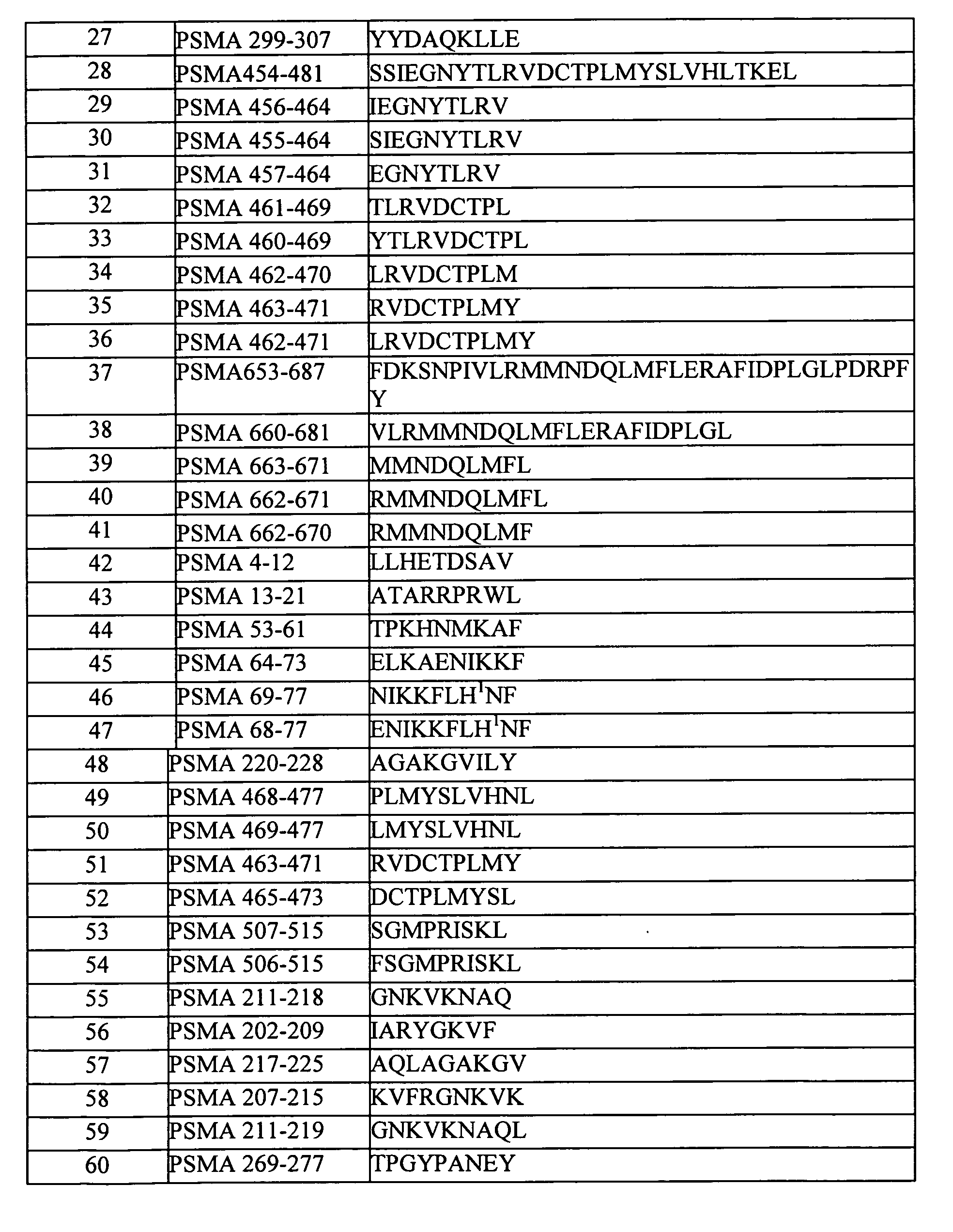

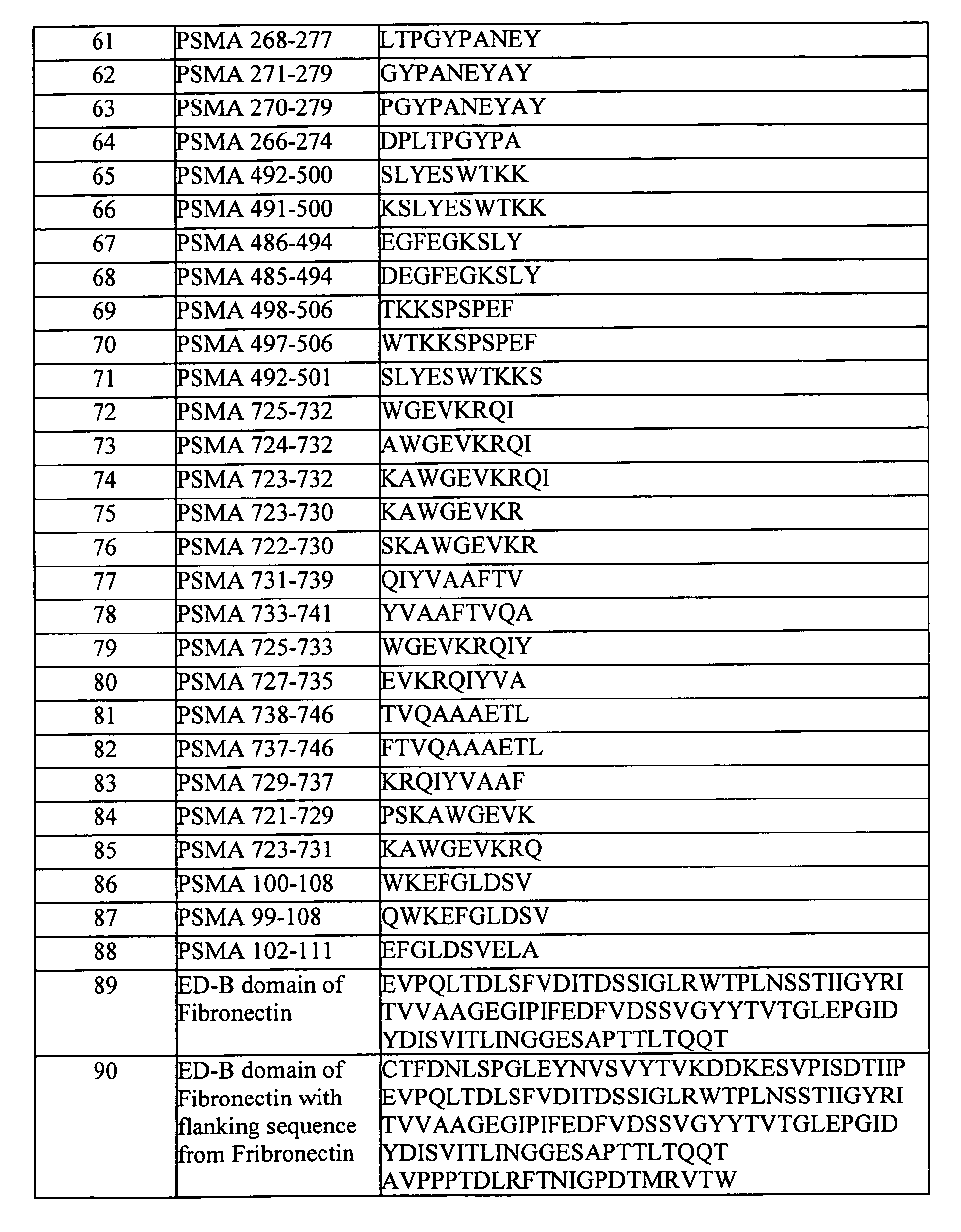

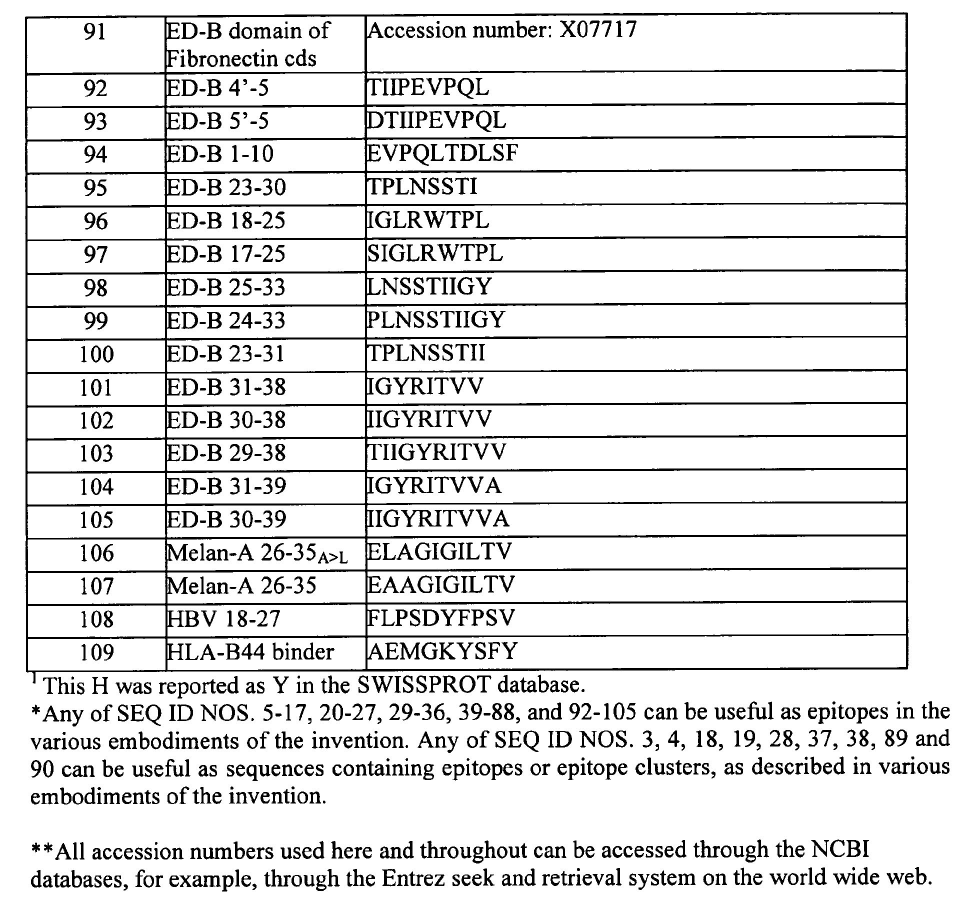

PSMA
LOCUS       NM_004476    2653 bp    mRNA          PRI       01-NOV-2000
DEFINITION  Homo sapiens folate hydrolase (prostate-specific membrane antigen)
            1 (FOLH1), mRNA.
ACCESSION   NM_004476
VERSION     NM_004476.1  GI:4758397
KEYWORDS    .
SOURCE      human.

ORGANISM Homo sapiens
Eukaryota; Metazoa; Chordata; Craniata; Vertebrata; Euteleostomi;
Mammalia; Eutheria; Primates; Catarrhini; Hominidae; Homo.
REFERENCE 1 (bases 1 to 2653)
AUTHORS Israeli,R.S., Powell,C.T., Fair,W.R. and Heston,W.D.
TITLE Molecular cloning of a complementary DNA encoding a
prostate-specific membrane antigen
JOURNAL Cancer Res. 53 (2), 227-230 (1993)
MEDLINE 93113576
REFERENCE 2 (bases 1 to 2653)
AUTHORS Rinker-Schaeffer CW, Hawkins AL, Su SL, Israeli RS, Griffin CA,
Isaacs JT and Heston WD.
TITLE Localization and physical mapping of the prostate-specific membrane
antigen (PSM) gene to human chromosome 11
JOURNAL Genomics 30 (1), 105-108 (1995)
MEDLINE 96129312
PUBMED 8595888
REFERENCE 3 (bases 1 to 2653)
AUTHORS O'Keefe DS, Su SL, Bacich DJ, Horiguchi Y, Luo Y, Powell CT,
Zandvliet D, Russell PJ, Molloy PL, Nowak NJ, Shows TB, Mullins C,
Vonder Haar RA, Fair WR and Heston WD.
TITLE Mapping, genomic organization and promoter analysis of the human
prostate-specific membrane antigen gene
JOURNAL Biochim. Biophys. Acta 1443 (1-2), 113-127 (1998)
MEDLINE 99057588
PUBMED 9838072
REFERENCE 4 (bases 1 to 2653)
AUTHORS Maraj BH, Leek JP, Karayi M, Ali M, Lench NJ and Markham AF.
TITLE Detailed genetic mapping around a putative prostate-specific
membrane antigen locus on human chromosome 11p11.2
JOURNAL Cytogenet. Cell Genet. 81 (1), 3-9 (1998)
MEDLINE 98358137
PUBMED 9691167
COMMENT PROVISIONAL REFSEQ: This record has not yet been subject to final
NCBI review. The reference sequence was derived from M99487.1.
FEATURES Location/Qualifiers
source 1..2653
/organism="Homo sapiens"
/db_xref="taxon:9606"
/chromosome="11"
/map="11p11.2"

/sex="male"
/cell_line="LNCaP-ATCC"
/cell_type="prostate"
/tissue_type="prostatic carcinoma metastatic lymph node"
/tissue_lib="LNCaP cDNA of Ron Israeli"
gene 1..2653
/gene="FOLH1"
/note="FOLH; PSM; PSMA"
/db_xref="LocusID:2346"
/db_xref="MIM:600934"
CDS 262..2514
/gene="FOLH1"
/note="folate hydrolase 1 (prostate-specific membrane
antigen)"
/codon_start=1
/db_xref="LocusID:2346"
/db_xref="MIM:600934"
/evidence=experimental
/product="folate hydrolase (prostate-specific membrane
antigen) 1"
/protein_id="NP_004467.1"
/db_xref="GI:4758398"

/translation="MWNLLHETDSAVATARRPRWLCAGALVLAGGFFLLGFLFGWFIK
SSNEATNITPKHNMKAFLDELKAENIKKFLYNFTQIPHLAGTEQNFQLAKQIQSQWKE
FGLDSVELAHYDVLLSYPNKTHPNYISIINEDGNEIFNTSLFEPPPPGYENVSDIVPP
FSAFSPQGMPEGDLVYVNYARTEDFFKLERDMKINCSGKIVIARYGKVFRGNKVKNA
Q
LAGAKGVILYSDPADYFAPGVKSYPDGWNLPGGGVQRGNILNLNGAGDPLTPGYPA
NE
YAYRRGIAEAVGLPSIPVHPIGYYDAQKLLEKMGGSAPPDSSWRGSLKVPYNVGPGF
T
GNFSTQKVKMHIHSTNEVTRIYNVIGTLRGAVEPDRYVILGGHRDSWVFGGIDPQSG
A
AVVHEIVRSFGTLKKEGWRPRRTILFASWDAEEFGLLGSTEWAEENSRLLQERGVAY
I
NADSSIEGNYTLRVDCTPLMYSLVHNLTKELKSPDEGFEGKSLYESWTKKSPSPEFSG
MPRISKLGSGNDFEVFFQRLGIASGRARYTKNWETNKFSGYPLYHSVYETYELVEKF
Y
DPMFKYHLTVAQVRGGMVFELANSIVLPFDCRDYAVVLRKYADKIYSISMKHPQEM
KT

YSVSFDSLFSAVKNFTEIASKFSERLQDFDKSNPIVLRMMNDQLMFLERAFIDPLGLP
DRPFYRHVIYAPSSHNKYAGESFPGIYDALFDIESKVDPSKAWGEVKRQIYVAAFTVQ
AAAETLSEVA" (SEQ ID NO. 1)
misc_feature 778..1029
/note="PA; Region: PA domain"
BASE COUNT 782 a 524 c 640 g 707 t
ORIGIN
1 ctcaaaaggg gccggatttc cttctcctgg aggcagatgt tgcctctctc tctcgctcgg
61 attggttcag tgcactctag aaacactgct gtggtggaga aactggaccc caggtctgga
121 gcgaattcca gcctgcaggg ctgataagcg aggcattagt gagattgaga gagactttac
181 cccgccgtgg tggttggagg gcgcgcagta gagcagcagc acaggcgcgg gtcccgggag
241 gccggctctg ctcgcgccga gatgtggaat ctccttcacg aaaccgactc ggctgtggcc
301 accgcgcgcc gcccgcgctg gctgtgcgct ggggcgctgg tgctggcggg tggcttcttt
361 ctcctcggct tcctcttcgg gtggtttata aaatcctcca atgaagctac taacattact
421 ccaaagcata atatgaaagc atttttggat gaattgaaag ctgagaacat caagaagttc
481 ttatataatt ttacacagat accacattta gcaggaacag aacaaaactt tcagcttgca
541 aagcaaattc aatcccagtg gaaagaattt ggcctggatt ctgttgagct agcacattat
601 gatgtcctgt tgtcctaccc aaataagact catcccaact acatctcaat aattaatgaa
661 gatggaaatg agattttcaa cacatcatta tttgaaccac ctcctccagg atatgaaaat
721 gtttcggata ttgtaccacc tttcagtgct ttctctcctc aaggaatgcc agagggcgat
781 ctagtgtatg ttaactatgc acgaactgaa gacttcttta aattggaacg ggacatgaaa
841 atcaattgct ctgggaaaat tgtaattgcc agatatggga aagttttcag aggaaataag
901 gttaaaaatg cccagctggc aggggccaaa ggagtcattc tctactccga ccctgctgac
961 tactttgctc ctggggtgaa gtcctatcca gatggttgga atcttcctgg aggtggtgtc
1021 cagcgtggaa atatcctaaa tctgaatggt gcaggagacc ctctcacacc aggttaccca
1081 gcaaatgaat atgcttatag gcgtggaatt gcagaggctg ttggtcttcc aagtattcct
1141 gttcatccaa ttggatacta tgatgcacag aagctcctag aaaaaatggg tggctcagca
1201 ccaccagata gcagctggag aggaagtctc aaagtgccct acaatgttgg acctggcttt
1261 actggaaact tttctacaca aaaagtcaag atgcacatcc actctaccaa tgaagtgaca
1321 agaatttaca atgtgatagg tactctcaga ggagcagtgg aaccagacag atatgtcatt
1381 ctgggaggtc accgggactc atgggtgttt ggtggtattg accctcagag tggagcagct
1441 gttgttcatg aaattgtgag gagctttgga acactgaaaa aggaagggtg gagacctaga
1501 agaacaattt tgtttgcaag ctgggatgca gaagaatttg gtcttcttgg ttctactgag
1561 tgggcagagg agaattcaag actccttcaa gagcgtggcg tggcttatat taatgctgac
1621 tcatctatag aaggaaacta cactctgaga gttgattgta caccgctgat gtacagcttg
1681 gtacacaacc taacaaaaga gctgaaaagc cctgatgaag gctttgaagg caaatctctt
1741 tatgaaagtt ggactaaaaa aagtccttcc ccagagttca gtggcatgcc caggataagc
1801 aaattgggat ctggaaatga ttttgaggtg ttcttccaac gacttggaat tgcttcaggc
1861 agagcacggt atactaaaaa ttgggaaaca aacaaattca gcggctatcc actgtatcac
1921 agtgtctatg aaacatatga gttggtggaa aagttttatg atccaatgtt taaatatcac
1981 ctcactgtgg cccaggttcg aggagggatg gtgtttgagc tagccaattc catagtgctc

2041 ccttttgatt gtcgagatta tgctgtagtt ttaagaaagt atgctgacaa aatctacagt
2101 atttctatga aacatccaca ggaaatgaag acatacagtg tatcatttga ttcacttttt
2161 tctgcagtaa agaattttac agaaattgct tccaagttca gtgagagact ccaggacttt
2221 gacaaaagca acccaatagt attaagaatg atgaatgatc aactcatgtt tctggaaaga
2281 gcatttattg atccattagg gttaccagac aggccttttt ataggcatgt catctatgct
2341 ccaagcagcc acaacaagta tgcaggggag tcattcccag gaatttatga tgctctgttt
2401 gatattgaaa gcaaagtgga cccttccaag gcctggggag aagtgaagag acagatttat
2461 gttgcagcct tcacagtgca ggcagctgca gagactttga gtgaagtagc ctaagaggat
2521 tctttagaga atccgtattg aatttgtgtg gtatgtcact cagaaagaat cgtaatgggt
2581 atattgataa attttaaaat tggtatattt gaaataaagt tgaatattat atataaaaaa
2641 aaaaaaaaaa aaa (SEQ ID NO. 2)

ED-B domain of Fibronectin
LOCUS HSFIBEDB 2823 bp DNA linear PRI 09-AUG-1999
DEFINITION Human fibronectin gene ED-B region.
ACCESSION X07717
VERSION X07717.1 GI:31406
KEYWORDS alternate splicing; fibronectin.
SOURCE human.
ORGANISM Homo sapiens
Eukaryota; Metazoa; Chordata; Craniata; Vertebrata; Euteleostomi;
Mammalia; Eutheria; Primates; Catarrhini; Hominidae; Homo.
REFERENCE 1 (bases 1 to 2823)
AUTHORS Paolella,G., Henchcliffe,C., Sebastio,G. and Baralle,F.E.
TITLE Sequence analysis and in vivo expression show that alternative
splicing of ED-B and ED-A regions of the human fibronectin gene are
independent events
JOURNAL Nucleic Acids Res. 16 (8), 3545-3557 (1988)
MEDLINE 88233940
FEATURES Location/Qualifiers

source 1..2823
/organism="Homo sapiens"
/db_xref="taxon:9606"
/clone="MA10"
exon 1..104
/number=1
/product="fibronectin"
CDS join(<2..104,1375..1647,2758..>2823)
/codon_start=1
/product="fibronectin"
/protein_id="CAB52437.1"
/db_xref="GI:5725425"

/translation="CTFDNLSPGLEYNVSVYTVKDDKESVPISDTIIPEVPQLTDLSF
VDITDSSIGLRWTPLNSSTIIGYRITVVAAGEGIPIFEDFVDSSVGYYTVTGLEPGID
YDISVITLINGGESAPTTLTQQTAVPPPTDLRFTNIGPDTMRVTW" (SEQ ID NO. 90)
intron 105..1374
/number=1
exon 1375..1647
/note="ED-B exon"
/number=2
/product="fibronectin"
intron 1648..2757
/number=2
exon 2758..2823
/number=3
/product="fibronectin"
BASE COUNT 824 a 556 c 528 g 915 t

ORIGIN
1 ctgcactttt gataacctga gtcccggcct ggagtacaat gtcagtgttt acactgtcaa
61 ggatgacaag gaaagtgtcc ctatctctga taccatcatc ccaggtaata gaaaataagc
121 tgctatcctg agagtgacat tccaataaga gtggggatta gcatcttaat ccccagatgc
181 ttaagggtgt caactatatt tgggatttaa ttccgatctc ccagctgcac tttccaaaac
241 caagaagtca aagcagcgat ttggacaaaa tgcttgctgt taacactgct ttactgtctg
301 tgcttcactg ggatgctgtg tgttgcagcg agtatgtaat ggagtggcag ccatggcttt
361 aactctgtat tgtctgctca catggaagta tgactaaaac actgtcacgt gtctgtactc
421 agtactgata ggctcaaagt aatatggtaa atgcatccca tcagtacatt tctgcccgat
481 tttacaatcc atatcaattt ccaacagctg cctatttcat cttgcagttt caaatccttc
541 tttttgaaaa ttggatttta aaaaaaagtt aagtaaaagt cacaccttca gggttgttct
601 ttcttgtggc cttgaaagac aacattgcaa aggcctgtcc taaggatagg cttgtttgtc
661 cattgggtta taacataatg aaagcattgg acagatcgtg tccccctttg gactcttcag
721 tagaatgctt ttactaacgc taattacatg ttttgattat gaatgaacct aaaatagtgg
781 caatggcctt aacctaggcc tgtctttcct cagcctgaat gtgcttttga atggcacatt
841 tcacaccata cattcataat gcattagcgt tatggccatg atgttgtcat gagttttgta
901 tgggagaaaa aaaatcaatt tatcacccat ttattatttt ttccggttgt tcatgcaagc
961 ttattttcta ctaaaacagt tttggaatta ttaaaagcat tgctgatact tacttcagat
1021 attatgtcta ggctctaaga atggtttcga catcctaaac agccatatga tttttaggaa
1081 tctgaacagt tcaaattgta ccctttaagg atgttttcaa aatgtaaaaa atatatatat
1141 atatatatat tccctaaaag aatattcctg tttattcttc tagggaagca aactgttcat
1201 gatgcttagg aagtcttttc agagaattta aaacagattg catattacca tcattgcttt
1261 aacattccac caattttact actagtaacc tgatatacac tgctttattt tttcctcttt
1321 ttttccctct attttccttt tgcctccccc tccctttgct ttgtaactca atagaggtgc
1381 cccaactcac tgacctaagc tttgttgata taaccgattc aagcatcggc ctgaggtgga
1441 ccccgctaaa ctcttccacc attattgggt accgcatcac agtagttgcg gcaggagaag
1501 gtatccctat ttttgaagat tttgtggact cctcagtagg atactacaca gtcacagggc
1561 tggagccggg cattgactat gatatcagcg ttatcactct cattaatggc ggcgagagtg

1621 cccctactac actgacacaa caaacgggtg aattttgaaa acttctgcgt ttgagacata
1681 gatggtgttg catgctgcca ccagttactc cggttaaata tggatgtttc atgggggaag
1741 tcagcaattg gccaaagatt cagataggtg gaattggggg gataaggaat caaatgcatc
1801 tgctaaactg attggagaaa aacacatgca atatcttcag tacactctca tttaaaccac
1861 aagtagatat aaagcctaga gaaatacaga tgtctgctct gttaaatata aaatagcaaa
1921 tgttcattca atttgaagac ctagaatttt tcttcttaaa taccaaacac gaataccaaa
1981 ttgcgtaagt accaattgat aagaatatat caccaaaatg taccatcatg ctcttccttc
2041 taccctttga taaactctac catgctcctt ctttgtagct aaaaacccat caaaatttag
2101 ggtagagtgg atgggcattg ttttgaggta ggagaaaagt aaacttggga ccattctagg
2161 ttttgttgct gtcactaggt aaagaaacac ctctttaacc acagtctggg gacaagcatg
2221 caacatttta aaggttctct gctgtgcatg ggaaaagaaa catgctgaga accaatttgc
2281 atgaacatgt tcacttgtaa gtagaattca ctgaatggaa ctgtagctct agatatctca
2341 catgggggga agtttaggac cctcttgtct ttttgtctgt gtgcatgtat ttctttgtaa
2401 agtactgcta tgtttctctt tgctgtgtgg caacttaagc ctcttcggcc tgggataaaa
2461 taatctgcag tggtattaat aatgtacata aagtcaacat atttgaaagt agattaaaat
2521 cttttttaaa tatatcaatg atggcaaaaa ggttaaaggg ggcctaacag tactgtgtgt
2581 agtgttttat ttttaacagt agtacactat aacttaaaat agacttagat tagactgttt
2641 gcatgattat gattctgttt cctttatgca tgaaatattg attttacctt tccagctact
2701 tcgttagctt taattttaaa atacattaac tgagtcttcc ttcttgttcg aaaccagctg
2761 ttcctcctcc cactgacctg cgattcacca acattggtcc agacaccatg cgtgtcacct
2821 ggg (SEQ ID NO. 91)
//

### Brief Description of the Drawings

Figure 1A, B, and C show results of N-terminal pool sequencing of a T=60 min. time point aliquot of the PSMA₁₆₃₋₁₉₂ proteasomal digest.
Figure 2 shows binding curves for HLA-A2:PSMA₁₆₈₋₁₇₇ and HLA-A2:PSMA₂₈₈₋₂₉₇ with controls.
Figure 3 shows results of N-terminal pool sequencing of a T=60 min. time point aliquot of the PSMA₂₈₁₋₃₁₀ proteasomal digest.
Figure 4 shows binding curves for HLA-A2:PSMA₄₆₁₋₄₆₉, HLA-A2:PSMA₄₆₀₋₄₆₉, and HLA-A2:PSMA₆₆₃₋₆₇₁, with controls.
Figure 5 shows the results of a □ (gamma)-IFN-based ELISPOT assay detecting PSMA₄₆₃₋₄₇₁-reactive HLA-A1⁺ CD8⁺ T cells.
Figure 6 shows blocking of reactivity of the T cells used in figure 10 by anti-HLA-A1 mAb, demonstrating HLA-A1-restricted recognition.
Figure 7 shows a binding curve for HLA-A2:PSMA₆₆₃₋₆₇₁, with controls.
Figure 8 shows a binding curve for HLA-A2:PSMA₆₆₂₋₆₇₁, with controls.
Figure 9 shows epitope specific lysis by CTL from HHD-A2 mice immunized with ED-B 29-38 peptide.

## Claims

1. A method of treating neoplastic disease comprising the step of immunizing a mammal to induce a cellular immune response directed against an antigen differentially expressed by tumor-associated neovasculature.

2. The method of claim 1, wherein the differentially expressed antigen is a protein.

3. The method of claim 2, wherein the protein is prostate specific membrane antigen.

4. The method of claim 1, wherein the antigen is the ED-B domain of fibronectin.

5. The method of claim 2, wherein immunization is carried out with at least one peptide derived from the sequence of said protein.

6. The method of claim 2, wherein immunization is carried out with a nucleic acid capable of conferring expression of said protein or peptides.

7. The method of claim 6, wherein the at least one peptide comprises a housekeeping epitope.

8. The method of claim 7, wherein the at least one peptide is co-C-terminal with the housekeeping epitope.

9. The method of claim 7, further comprising at least one additional peptide wherein the at least one additional peptide comprises an immune epitope.

10. The method of claim 1, comprising an additional step wherein the mammal is treated with an anti-tumor therapy active directly against cancerous cells.

11. The method of claim 10, wherein the anti-tumor therapy is immunization against a tumor-associated antigen.

12. The method of claim 1, wherein the cellular immune response comprises a CTL response.

13. An immunogenic composition comprising at least one immunogen corresponding to an antigen expressed by tumor-associated neovasculature, wherein the composition can induce a cellular immune response.

14. The composition of claim 13, wherein the immunogen is not associated with a cell conspecific with the recipient.

15. The composition of claim 13, wherein the antigen is a protein.

16. The composition of claim 15, wherein the protein is prostate specific membrane antigen.

17. The composition of claim 13, wherein the antigen is the ED-B domain of fibronectin.

18. The composition of claim 13, wherein the immunogen comprises at least one peptide.

19. The composition of claim 13, comprising a nucleic acid capable of conferring expression of said antigen, and wherein said antigen is a protein or a peptide.

20. The composition of claim 19, comprising at least one peptide comprising a housekeeping epitope.

21. The composition of claim 20, wherein the at least one peptide is co-C-terminal with the housekeeping epitope.

22. The composition of claim 20, additionally comprising at least one peptide comprising an immune epitope.

23. The composition of claim 13, additionally comprising at least one immunogen corresponding to a tumor-associated antigen.

24. The composition of claim 13, wherein the cellular immune response comprises a CTL response.

25. A method of evaluating cell-mediated immunity, comprising the steps of:
- implanting or injecting MHC-transgenic tumor cells into an MHC-transgenic mammal;
- establishing an immune response in the mammal; and
- assaying a characteristic to determine cell-mediated immunity in the mammal.

26. The method of claim 25, wherein the MHC-transgenic mammal is an HLA-transgenic mammal.

27. The method of claim 26, wherein the HLA-transgenic mammal is an HLA-A2 transgenic mammal.

28. The method of claim 25, wherein the mammal is a mouse.

29. The method of claim 25, wherein the cell-mediated immunity is established by vaccination.

30. The method of claim 29, wherein the vaccination takes place prior to, concurrent with, or subsequent to transfer of the tumor cells.

31. The method of claim 25, wherein the cell-mediated immunity is mediated by cytotoxic T lymphocytes.

32. The method of claim 25, wherein the neovasculature antigen is preferentially expressed by tumor-associated neovasculature.

33. The method of claim 25, wherein the neovasculature antigen is also a tumor-associated antigen.

34. The method of claim 33, wherein the antigen is the ED-B domain of fibronectin.

35. The method of claim 25, wherein the characterizing step comprises assessing a parameter selected from the group consisting of tumor presence, tumor growth, tumor size, rapidity of tumor appearance, dose of vaccine required to inhibit or prevent tumor establishment, tumor vascularization, and a proportion of necrotic tissue within the tumor.

36. The method of claim 25, further comprising the steps of:
- providing a first population of mammals and a second populations of mammals;
- establishing cell-mediated immunity in the first population;
- differentially establishing cell-mediated immunity in the second population; and
- comparing a result obtained from the first population of mammals to a result obtained from the second population of mammals.

37. The method of claim 36, wherein the cell-mediated immunity of the first population is selected from the group consisting of naive immunity and immunity to an irrelevant epitope.

38. A method of anti-tumor vaccine design comprising the steps of:
identifying an antigen differentially expressed by tumor-associated neovasculature; and
incorporating a component of the antigen into a vaccine.

39. The method of claim 38, wherein the component is a polypeptide fragment of the antigen.

40. The method of claim 38, wherein the component is a nucleic acid encoding the antigen or a fragment of the antigen.

41. A method of making a research model, comprising implanting a vascular cell and a tumor cell into an immunodeficient mammal, wherein said tumor cell is implanted adjacent to said vascular cell.

42. The method of claim 41, wherein said vascular cell is a vascular endothelial cell.

43. The method of claim 42, wherein said vascular endothelial cell is HDMEC.

44. The method of claim 42, wherein said vascular endothelial cell is telomerase-transformed.

45. The method of claim 41, wherein said immunodeficient mammal is a mouse.

46. The method of claim 45, wherein said mouse is a SCID mouse.

47. A research model comprising an immunodeficient mammal, the mammal comprising an implanted vascular cell and an implanted tumor cell, wherein said vascular cell and said tumor cell are implanted adjacent to one another.

48. A method of evaluating cell-mediated immunity comprising the steps of:
- implanting vascular cells into an immunodeficient mammal;
- establishing an immune response in the mammal; and
- assaying a characteristic to determine cell-mediated immunity in the mammal.

49. The method of claim 48, wherein the cell-mediated immunity is directed against a neovasculature antigen.

50. The method of claim 49, wherein the neovasculature antigen is preferentially expressed by tumor-associated neovasculature.

51. The method of claim 49, wherein the neovasculature antigen is prostate specific membrane antigen (PSMA) or vascular endothelial growth factor receptor 2 (VEGFR2).

52. The method of claim 48, wherein the establishing step is achieved by adoptive transfer of T-cells to the mammal.

53. The method of claim 48, wherein the establishing step is achieved by contacting the mammal with an antigen.

54. The method of claim 48, wherein the cell-mediated immunity is mediated by cytotoxic T lymphocytes.

55. The method of claim 48, wherein the vascular cells are vascular endothelial cells.

56. The method of claim 55, wherein the endothelial cells are HDMEC.

57. The method of claim 55, wherein the endothelial cells are telomerase-transformed endothelial cells.

58. The method of claim 48, wherein the immunodeficient mammal is a mouse.

59. The method of claim 58, wherein the immunodeficient mouse is a SCID mouse.

60. The method of claim 48, wherein the characterizing step comprises assessing a parameter selected from the group consisting of vessel formation, vessel destruction, vessel density, and proportion of vessels carrying blood of the host mammal.

61. The method of claim 48, further comprising the step of implanting tumor cells or tumor tissue in the mouse.

62. The method of claim 61, wherein the characterizing step comprises assessing a parameter selected from the group consisting of tumor presence, tumor growth, tumor size, rapidity of tumor appearance, dose of vaccine required to inhibit or prevent tumor establishment, tumor vascularization, and a proportion of necrotic tissue within the tumor.

63. The method of claim 48, further comprising the steps of:
- providing a first population of mammals and a second populations of mammals;
- establishing cell-mediated immunity in the first population;
- differentially establishing cell-mediated immunity in the second population; and
- comparing a result obtained from the first population of mammals to a result obtained from the second population of mammals.

64. The method of claim 63, wherein the cell-mediated immunity of the first population is selected from the group consisting of naive immunity and immunity to an irrelevant epitope.


SEQUENCE LISTING

<110> MANNKIND CORPORATION

<120> ANTI-NEOVASCULATURE PREPARATIONS FOR
  CANCER

<130> M 9369/RN

<141> 2002-03-07

<150> US 60/274,063
<151> 2001-03-07

<160> 159

<170> FastSEQ for Windows Version 4.0

<210> 1
<211> 750
<212> PRT
<213> Homo sapiens

<400> 1
Met Trp Asn Leu Leu His Glu Thr Asp Ser Ala Val Ala Thr Ala Arg
 1               5                  10                  15
Arg Pro Arg Trp Leu Cys Ala Gly Ala Leu Val Leu Ala Gly Gly Phe
            20                  25                  30
Phe Leu Leu Gly Phe Leu Phe Gly Trp Phe Ile Lys Ser Ser Asn Glu
        35                  40                  45
Ala Thr Asn Ile Thr Pro Lys His Asn Met Lys Ala Phe Leu Asp Glu
    50                  55                  60
Leu Lys Ala Glu Asn Ile Lys Lys Phe Leu Tyr Asn Phe Thr Gln Ile
65                  70                  75                  80
Pro His Leu Ala Gly Thr Glu Gln Asn Phe Gln Leu Ala Lys Gln Ile
                85                  90                  95
Gln Ser Gln Trp Lys Glu Phe Gly Leu Asp Ser Val Glu Leu Ala His
            100                 105                 110
Tyr Asp Val Leu Leu Ser Tyr Pro Asn Lys Thr His Pro Asn Tyr Ile
        115                 120                 125
Ser Ile Ile Asn Glu Asp Gly Asn Glu Ile Phe Asn Thr Ser Leu Phe
    130                 135                 140
Glu Pro Pro Pro Pro Gly Tyr Glu Asn Val Ser Asp Ile Val Pro Pro
145                 150                 155                 160
Phe Ser Ala Phe Ser Pro Gln Gly Met Pro Glu Gly Asp Leu Val Tyr
                165                 170                 175
Val Asn Tyr Ala Arg Thr Glu Asp Phe Phe Lys Leu Glu Arg Asp Met
            180                 185                 190
Lys Ile Asn Cys Ser Gly Lys Ile Val Ile Ala Arg Tyr Gly Lys Val
        195                 200                 205
Phe Arg Gly Asn Lys Val Lys Asn Ala Gln Leu Ala Gly Ala Lys Gly
    210                 215                 220
Val Ile Leu Tyr Ser Asp Pro Ala Asp Tyr Phe Ala Pro Gly Val Lys
225                 230                 235                 240
Ser Tyr Pro Asp Gly Trp Asn Leu Pro Gly Gly Gly Val Gln Arg Gly
                245                 250                 255
Asn Ile Leu Asn Leu Asn Gly Ala Gly Asp Pro Leu Thr Pro Gly Tyr
            260                 265                 270
Pro Ala Asn Glu Tyr Ala Tyr Arg Arg Gly Ile Ala Glu Ala Val Gly
        275                 280                 285
Leu Pro Ser Ile Pro Val His Pro Ile Gly Tyr Tyr Asp Ala Gln Lys
    290                 295                 300
Leu Leu Glu Lys Met Gly Gly Ser Ala Pro Pro Asp Ser Ser Trp Arg
305                 310                 315                 320
Gly Ser Leu Lys Val Pro Tyr Asn Val Gly Pro Gly Phe Thr Gly Asn
                325                 330                 335
Phe Ser Thr Gln Lys Val Lys Met His Ile His Ser Thr Asn Glu Val

            340                 345                 350
Thr Arg Ile Tyr Asn Val Ile Gly Thr Leu Arg Gly Ala Val Glu Pro
        355                 360                 365
Asp Arg Tyr Val Ile Leu Gly Gly His Arg Asp Ser Trp Val Phe Gly
    370                 375                 380
Gly Ile Asp Pro Gln Ser Gly Ala Ala Val Val His Glu Ile Val Arg
385                 390                 395                 400
Ser Phe Gly Thr Leu Lys Lys Glu Gly Trp Arg Pro Arg Arg Thr Ile
                405                 410                 415
Leu Phe Ala Ser Trp Asp Ala Glu Glu Phe Gly Leu Leu Gly Ser Thr
            420                 425                 430
Glu Trp Ala Glu Glu Asn Ser Arg Leu Leu Gln Glu Arg Gly Val Ala
        435                 440                 445
Tyr Ile Asn Ala Asp Ser Ser Ile Glu Gly Asn Tyr Thr Leu Arg Val
    450                 455                 460
Asp Cys Thr Pro Leu Met Tyr Ser Leu Val His Asn Leu Thr Lys Glu
465                 470                 475                 480
Leu Lys Ser Pro Asp Glu Gly Phe Glu Gly Lys Ser Leu Tyr Glu Ser
                485                 490                 495
Trp Thr Lys Lys Ser Pro Ser Pro Glu Phe Ser Gly Met Pro Arg Ile
            500                 505                 510
Ser Lys Leu Gly Ser Gly Asn Asp Phe Glu Val Phe Phe Gln Arg Leu
        515                 520                 525
Gly Ile Ala Ser Gly Arg Ala Arg Tyr Thr Lys Asn Trp Glu Thr Asn
    530                 535                 540
Lys Phe Ser Gly Tyr Pro Leu Tyr His Ser Val Tyr Glu Thr Tyr Glu
545                 550                 555                 560
Leu Val Glu Lys Phe Tyr Asp Pro Met Phe Lys Tyr His Leu Thr Val
                565                 570                 575
Ala Gln Val Arg Gly Gly Met Val Phe Glu Leu Ala Asn Ser Ile Val
            580                 585                 590
Leu Pro Phe Asp Cys Arg Asp Tyr Ala Val Val Leu Arg Lys Tyr Ala
        595                 600                 605
Asp Lys Ile Tyr Ser Ile Ser Met Lys His Pro Gln Glu Met Lys Thr
    610                 615                 620
Tyr Ser Val Ser Phe Asp Ser Leu Phe Ser Ala Val Lys Asn Phe Thr
625                 630                 635                 640
Glu Ile Ala Ser Lys Phe Ser Glu Arg Leu Gln Asp Phe Asp Lys Ser
                645                 650                 655
Asn Pro Ile Val Leu Arg Met Met Asn Asp Gln Leu Met Phe Leu Glu
            660                 665                 670
Arg Ala Phe Ile Asp Pro Leu Gly Leu Pro Asp Arg Pro Phe Tyr Arg
        675                 680                 685
His Val Ile Tyr Ala Pro Ser Ser His Asn Lys Tyr Ala Gly Glu Ser
    690                 695                 700
Phe Pro Gly Ile Tyr Asp Ala Leu Phe Asp Ile Glu Ser Lys Val Asp
705                 710                 715                 720
Pro Ser Lys Ala Trp Gly Glu Val Lys Arg Gln Ile Tyr Val Ala Ala
                725                 730                 735
Phe Thr Val Gln Ala Ala Ala Glu Thr Leu Ser Glu Val Ala
            740                 745                 750

<210> 2
<211> 2653
<212> DNA
<213> Homo sapiens

<400> 2
ctcaaaaggg gccggatttc cttctcctgg aggcagatgt tgcctctctc tctcgctcgg 60
attggttcag tgcactctag aaacactgct gtggtggaga aactggaccc caggtctgga 120
gcgaattcca gcctgcaggg ctgataagcg aggcattagt gagattgaga gagactttac 180
cccgccgtgg tggttggagg gcgcgcagta gagcagcagc acaggcgcgg gtcccgggag 240
gccggctctg ctcgcgccga gatgtggaat ctccttcacg aaaccgactc ggctgtggcc 300
accgcgcgcc gcccgcgctg gctgtgcgct ggggcgctgg tgctggcggg tggcttcttt 360
ctcctcggct tcctcttcgg gtggtttata aaatcctcca atgaagctac taacattact 420
ccaaagcata atatgaaagc atttttggat gaattgaaag ctgagaacat caagaagttc 480
ttatataatt ttacacagat accacattta gcaggaacag aacaaaactt tcagcttgca 540

aagcaaattc aatcccagtg gaaagaattt ggcctggatt ctgttgagct agcacattat 600
gatgtcctgt tgtcctaccc aaataagact catcccaact acatctcaat aattaatgaa 660
gatggaaatg agattttcaa cacatcatta tttgaaccac ctcctccagg atatgaaaat 720
gtttcggata ttgtaccacc tttcagtgct ttctctcctc aaggaatgcc agagggcgat 780
ctagtgtatg ttaactatgc acgaactgaa gacttcttta aattggaacg ggacatgaaa 840
atcaattgct ctgggaaaat tgtaattgcc agatatggga aagttttcag aggaaataag 900
gttaaaaatg cccagctggc aggggccaaa ggagtcattc tctactccga ccctgctgac 960
tactttgctc ctggggtgaa gtcctatcca gatggttgga atcttcctgg aggtggtgtc 1020
cagcgtggaa atatcctaaa tctgaatggt gcaggagacc ctctcacacc aggttaccca 1080
gcaaatgaat atgcttatag gcgtggaatt gcagaggctg ttggtcttcc aagtattcct 1140
gttcatccaa ttggatacta tgatgcacag aagctcctag aaaaaatggg tggctcagca 1200
ccaccagata gcagctggag aggaagtctc aaagtgccct acaatgttgg acctggcttt 1260
actggaaact tttctacaca aaaagtcaag atgcacatcc actctaccaa tgaagtgaca 1320
agaatttaca atgtgatagg tactctcaga ggagcagtgg aaccagacag atatgtcatt 1380
ctgggaggtc accgggactc atgggtgttt ggtggtattg accctcagag tggagcagct 1440
gttgttcatg aaattgtgag gagctttgga acactgaaaa aggaagggtg gagacctaga 1500
agaacaattt tgtttgcaag ctgggatgca gaagaatttg gtcttcttgg ttctactgag 1560
tgggcagagg agaattcaag actccttcaa gagcgtggcg tggcttatat taatgctgac 1620
tcatctatag aaggaaacta cactctgaga gttgattgta caccgctgat gtacagcttg 1680
gtacacaacc taacaaaaga gctgaaaagc cctgatgaag gctttgaagg caaatctctt 1740
tatgaaagtt ggactaaaaa aagtccttcc ccagagttca gtggcatgcc caggataagc 1800
aaattgggat ctggaaatga ttttgaggtg ttcttccaac gacttggaat tgcttcaggc 1860
agagcacggt atactaaaaa ttgggaaaca aacaaattca gcggctatcc actgtatcac 1920
agtgtctatg aaacatatga gttggtggaa aagttttatg atccaatgtt taaatatcac 1980
ctcactgtgg cccaggttcg aggagggatg gtgtttgagc tagccaattc catagtgctc 2040
ccttttgatt gtcgagatta tgctgtagtt ttaagaaagt atgctgacaa aatctacagt 2100
atttctatga aacatccaca ggaaatgaag acatacagtg tatcatttga ttcacttttt 2160
tctgcagtaa agaattttac agaaattgct tccaagttca gtgagagact ccaggacttt 2220
gacaaaagca acccaatagt attaagaatg atgaatgatc aactcatgtt tctggaaaga 2280
gcatttattg atccattagg gttaccagac aggccttttt ataggcatgt catctatgct 2340
ccaagcagcc acaacaagta tgcaggggag tcattcccag gaatttatga tgctctgttt 2400
gatattgaaa gcaaagtgga cccttccaag gcctggggag aagtgaagag acagatttat 2460
gttgcagcct tcacagtgca ggcagctgca gagactttga gtgaagtagc ctaagaggat 2520
tctttagaga atccgtattg aatttgtgtg gtatgtcact cagaaagaat cgtaatgggt 2580
atattgataa attttaaaat tggtatattt gaaataaagt tgaatattat atataaaaaa 2640
aaaaaaaaaa aaa 2653

<210> 3
<211> 30
<212> PRT
<213> Homo sapiens

<400> 3
Ala Phe Ser Pro Gln Gly Met Pro Glu Gly Asp Leu Val Tyr Val Asn
1               5                   10                  15
Tyr Ala Arg Thr Glu Asp Phe Phe Lys Leu Glu Arg Asp Met
            20                  25                  30

<210> 4
<211> 23
<212> PRT
<213> Homo sapiens

<400> 4
Gly Met Pro Glu Gly Asp Leu Val Tyr Val Asn Tyr Ala Arg Thr Glu
1               5                   10                  15
Asp Phe Phe Lys Leu Glu Arg
            20

<210> 5
<211> 9
<212> PRT
<213> Homo sapiens

<400> 5
Met Pro Glu Gly Asp Leu Val Tyr Val

1                   5


<210> 6
<211> 10
<212> PRT
<213> Homo sapiens

<400> 6
Gly Met Pro Glu Gly Asp Leu Val Tyr Val
1                   5                   10


<210> 7
<211> 9
<212> PRT
<213> Homo sapiens

<400> 7
Gly Met Pro Glu Gly Asp Leu Val Tyr
1                   5


<210> 8
<211> 10
<212> PRT
<213> Homo sapiens

<400> 8
Gln Gly Met Pro Glu Gly Asp Leu Val Tyr
1                   5                   10


<210> 9
<211> 8
<212> PRT
<213> Homo sapiens

<400> 9
Met Pro Glu Gly Asp Leu Val Tyr
1                   5


<210> 10
<211> 9
<212> PRT
<213> Homo sapiens

<400> 10
Glu Gly Asp Leu Val Tyr Val Asn Tyr
1                   5


<210> 11
<211> 10
<212> PRT
<213> Homo sapiens

<400> 11
Pro Glu Gly Asp Leu Val Tyr Val Asn Tyr
1                   5                   10


<210> 12
<211> 10
<212> PRT
<213> Homo sapiens

<400> 12
Leu Val Tyr Val Asn Tyr Ala Arg Thr Glu
1               5                   10


<210> 13
<211> 9
<212> PRT
<213> Homo sapiens

<400> 13
Val Asn Tyr Ala Arg Thr Glu Asp Phe
1               5


<210> 14
<211> 10
<212> PRT
<213> Homo sapiens

<400> 14
Tyr Val Asn Tyr Ala Arg Thr Glu Asp Phe
1               5                   10


<210> 15
<211> 9
<212> PRT
<213> Homo sapiens

<400> 15
Asn Tyr Ala Arg Thr Glu Asp Phe Phe
1               5


<210> 16
<211> 8
<212> PRT
<213> Homo sapiens

<400> 16
Tyr Ala Arg Thr Glu Asp Phe Phe
1               5


<210> 17
<211> 9
<212> PRT
<213> Homo sapiens

<400> 17
Arg Thr Glu Asp Phe Phe Lys Leu Glu
1               5


<210> 18
<211> 30
<212> PRT
<213> Homo sapiens

<400> 18
Arg Gly Ile Ala Glu Ala Val Gly Leu Pro Ser Ile Pro Val His Pro
1               5                   10                  15
Ile Gly Tyr Tyr Asp Ala Gln Lys Leu Leu Glu Lys Met Gly
            20                  25                  30


<210> 19

<211> 25
<212> PRT
<213> Homo sapiens

<400> 19
Ile Ala Glu Ala Val Gly Leu Pro Ser Ile Pro Val His Pro Ile Gly
1               5                   10                  15
Tyr Tyr Asp Ala Gln Lys Leu Leu Glu
            20                  25


<210> 20
<211> 9
<212> PRT
<213> Homo sapiens

<400> 20
Leu Pro Ser Ile Pro Val His Pro Ile
1               5


<210> 21
<211> 10
<212> PRT
<213> Homo sapiens

<400> 21
Gly Leu Pro Ser Ile Pro Val His Pro Ile
1               5                   10


<210> 22
<211> 9
<212> PRT
<213> Homo sapiens

<400> 22
Ile Gly Tyr Tyr Asp Ala Gln Lys Leu
1               5


<210> 23
<211> 10
<212> PRT
<213> Homo sapiens

<400> 23
Pro Ile Gly Tyr Tyr Asp Ala Gln Lys Leu
1               5                   10


<210> 24
<211> 9
<212> PRT
<213> Homo sapiens

<400> 24
Ser Ile Pro Val His Pro Ile Gly Tyr
1               5


<210> 25
<211> 10
<212> PRT
<213> Homo sapiens

<400> 25
Pro Ser Ile Pro Val His Pro Ile Gly Tyr

1                   5                   10


<210> 26
<211> 8
<212> PRT
<213> Homo sapiens

<400> 26
Ile Pro Val His Pro Ile Gly Tyr
1               5


<210> 27
<211> 9
<212> PRT
<213> Homo sapiens

<400> 27
Tyr Tyr Asp Ala Gln Lys Leu Leu Glu
1               5


<210> 28
<211> 27
<212> PRT
<213> Homo sapiens

<400> 28
Ser Ser Ile Glu Gly Asn Tyr Thr Leu Arg Val Asp Cys Thr Pro Leu
1               5                   10                  15
Met Tyr Ser Leu Val His Leu Thr Lys Glu Leu
            20                  25


<210> 29
<211> 9
<212> PRT
<213> Homo sapiens

<400> 29
Ile Glu Gly Asn Tyr Thr Leu Arg Val
1               5


<210> 30
<211> 10
<212> PRT
<213> Homo sapiens

<400> 30
Ser Ile Glu Gly Asn Tyr Thr Leu Arg Val
1               5                   10


<210> 31
<211> 8
<212> PRT
<213> Homo sapiens

<400> 31
Glu Gly Asn Tyr Thr Leu Arg Val
1               5


<210> 32
<211> 9
<212> PRT

<213> Homo sapiens

<400> 32
Thr Leu Arg Val Asp Cys Thr Pro Leu
1               5


<210> 33
<211> 10
<212> PRT
<213> Homo sapiens

<400> 33
Tyr Thr Leu Arg Val Asp Cys Thr Pro Leu
1               5                   10


<210> 34
<211> 9
<212> PRT
<213> Homo sapiens

<400> 34
Leu Arg Val Asp Cys Thr Pro Leu Met
1               5


<210> 35
<211> 9
<212> PRT
<213> Homo sapiens

<400> 35
Arg Val Asp Cys Thr Pro Leu Met Tyr
1               5


<210> 36
<211> 10
<212> PRT
<213> Homo sapiens

<400> 36
Leu Arg Val Asp Cys Thr Pro Leu Met Tyr
1               5                   10


<210> 37
<211> 35
<212> PRT
<213> Homo sapiens

<400> 37
Phe Asp Lys Ser Asn Pro Ile Val Leu Arg Met Met Asn Asp Gln Leu
1               5                   10                  15
Met Phe Leu Glu Arg Ala Phe Ile Asp Pro Leu Gly Leu Pro Asp Arg
            20                  25                  30
Pro Phe Tyr
        35


<210> 38
<211> 22
<212> PRT
<213> Homo sapiens

<400> 38
Val Leu Arg Met Met Asn Asp Gln Leu Met Phe Leu Glu Arg Ala Phe

1                   5                   10                  15
Ile Asp Pro Leu Gly Leu
            20

<210> 39
<211> 9
<212> PRT
<213> Homo sapiens

<400> 39
Met Met Asn Asp Gln Leu Met Phe Leu
 1               5

<210> 40
<211> 10
<212> PRT
<213> Homo sapiens

<400> 40
Arg Met Met Asn Asp Gln Leu Met Phe Leu
 1               5                   10

<210> 41
<211> 9
<212> PRT
<213> Homo sapiens

<400> 41
Arg Met Met Asn Asp Gln Leu Met Phe
 1               5

<210> 42
<211> 9
<212> PRT
<213> Homo sapiens

<400> 42
Leu Leu His Glu Thr Asp Ser Ala Val
 1               5

<210> 43
<211> 9
<212> PRT
<213> Homo sapiens

<400> 43
Ala Thr Ala Arg Arg Pro Arg Trp Leu
 1               5

<210> 44
<211> 9
<212> PRT
<213> Homo sapiens

<400> 44
Thr Pro Lys His Asn Met Lys Ala Phe
 1               5

<210> 45
<211> 10
<212> PRT

<213> Homo sapiens

<400> 45
Glu Leu Lys Ala Glu Asn Ile Lys Lys Phe
1               5                   10


<210> 46
<211> 9
<212> PRT
<213> Homo sapiens

<220>
<221> VARIANT
<222> (7)...(7)
<223> Xaa = His or Tyr

<400> 46
Asn Ile Lys Lys Phe Leu Xaa Asn Phe
1               5


<210> 47
<211> 10
<212> PRT
<213> Homo sapiens

<220>
<221> VARIANT
<222> (8)...(8)
<223> Xaa = His or Tyr

<400> 47
Glu Asn Ile Lys Lys Phe Leu Xaa Asn Phe
1               5                   10


<210> 48
<211> 9
<212> PRT
<213> Homo sapiens

<400> 48
Ala Gly Ala Lys Gly Val Ile Leu Tyr
1               5


<210> 49
<211> 10
<212> PRT
<213> Homo sapiens

<400> 49
Pro Leu Met Tyr Ser Leu Val His Asn Leu
1               5                   10


<210> 50
<211> 9
<212> PRT
<213> Homo sapiens

<400> 50
Leu Met Tyr Ser Leu Val His Asn Leu
1               5


<210> 51

<211> 9
<212> PRT
<213> Homo sapiens

<400> 51
Arg Val Asp Cys Thr Pro Leu Met Tyr
1               5


<210> 52
<211> 9
<212> PRT
<213> Homo sapiens

<400> 52
Asp Cys Thr Pro Leu Met Tyr Ser Leu
1               5


<210> 53
<211> 9
<212> PRT
<213> Homo sapiens

<400> 53
Ser Gly Met Pro Arg Ile Ser Lys Leu
1               5


<210> 54
<211> 10
<212> PRT
<213> Homo sapiens

<400> 54
Phe Ser Gly Met Pro Arg Ile Ser Lys Le
1               5                   10


<210> 55
<211> 8
<212> PRT
<213> Homo sapiens

<400> 55
Gly Asn Lys Val Lys Asn Ala Gln
1               5


<210> 56
<211> 8
<212> PRT
<213> Homo sapiens

<400> 56
Ile Ala Arg Tyr Gly Lys Val Phe
1               5


<210> 57
<211> 9
<212> PRT
<213> Homo sapiens

<400> 57
Ala Gln Leu Ala Gly Ala Lys Gly Val
1               5

<210> 58
<211> 9
<212> PRT
<213> Homo sapiens

<400> 58
Lys Val Phe Arg Gly Asn Lys Val Lys
1               5


<210> 59
<211> 9
<212> PRT
<213> Homo sapiens

<400> 59
Gly Asn Lys Val Lys Asn Ala Gln Leu
1               5


<210> 60
<211> 9
<212> PRT
<213> Homo sapiens

<400> 60
Thr Pro Gly Tyr Pro Ala Asn Glu Tyr
1               5


<210> 61
<211> 10
<212> PRT
<213> Homo sapiens

<400> 61
Leu Thr Pro Gly Tyr Pro Ala Asn Glu Tyr
1               5                   10


<210> 62
<211> 9
<212> PRT
<213> Homo sapiens

<400> 62
Gly Tyr Pro Ala Asn Glu Tyr Ala Tyr
1               5


<210> 63
<211> 10
<212> PRT
<213> Homo sapiens

<400> 63
Pro Gly Tyr Pro Ala Asn Glu Tyr Ala Tyr
1               5                   10


<210> 64
<211> 9
<212> PRT
<213> Homo sapiens

<400> 64
Asp Pro Leu Thr Pro Gly Tyr Pro Ala

1               5


<210> 65
<211> 9
<212> PRT
<213> Homo sapiens

<400> 65
Ser Leu Tyr Glu Ser Trp Thr Lys Lys
1               5


<210> 66
<211> 10
<212> PRT
<213> Homo sapiens

<400> 66
Lys Ser Leu Tyr Glu Ser Trp Thr Lys Lys
1               5                   10


<210> 67
<211> 9
<212> PRT
<213> Homo sapiens

<400> 67
Glu Gly Phe Glu Gly Lys Ser Leu Tyr
1               5


<210> 68
<211> 10
<212> PRT
<213> Homo sapiens

<400> 68
Asp Glu Gly Phe Glu Gly Lys Ser Leu Tyr
1               5                   10


<210> 69
<211> 9
<212> PRT
<213> Homo sapiens

<400> 69
Thr Lys Lys Ser Pro Ser Pro Glu Phe
1               5


<210> 70
<211> 10
<212> PRT
<213> Homo sapiens

<400> 70
Trp Thr Lys Lys Ser Pro Ser Pro Glu Phe
1               5                   10


<210> 71
<211> 10
<212> PRT
<213> Homo sapiens

<400> 71
Ser Leu Tyr Glu Ser Trp Thr Lys Lys Ser
1               5                   10

<210> 72
<211> 8
<212> PRT
<213> Homo sapiens

<400> 72
Trp Gly Glu Val Lys Arg Gln Ile
1               5

<210> 73
<211> 9
<212> PRT
<213> Homo sapiens

<400> 73
Ala Trp Gly Glu Val Lys Arg Gln Ile
1               5

<210> 74
<211> 10
<212> PRT
<213> Homo sapiens

<400> 74
Lys Ala Trp Gly Glu Val Lys Arg Gln Ile
1               5                   10

<210> 75
<211> 8
<212> PRT
<213> Homo sapiens

<400> 75
Lys Ala Trp Gly Glu Val Lys Arg
1               5

<210> 76
<211> 9
<212> PRT
<213> Homo sapiens

<400> 76
Ser Lys Ala Trp Gly Glu Val Lys Arg
1               5

<210> 77
<211> 9
<212> PRT
<213> Homo sapiens

<400> 77
Gln Ile Tyr Val Ala Ala Phe Thr Val
1               5

<210> 78
<211> 9
<212> PRT

<213> Homo sapiens

<400> 78
Tyr Val Ala Ala Phe Thr Val Gln Ala
1               5


<210> 79
<211> 9
<212> PRT
<213> Homo sapiens

<400> 79
Trp Gly Glu Val Lys Arg Gln Ile Tyr
1               5


<210> 80
<211> 9
<212> PRT
<213> Homo sapiens

<400> 80
Glu Val Lys Arg Gln Ile Tyr Val Ala
1               5


<210> 81
<211> 9
<212> PRT
<213> Homo sapiens

<400> 81
Thr Val Gln Ala Ala Ala Glu Thr Leu
1               5


<210> 82
<211> 10
<212> PRT
<213> Homo sapiens

<400> 82
Phe Thr Val Gln Ala Ala Ala Glu Thr Leu
1               5                   10


<210> 83
<211> 9
<212> PRT
<213> Homo sapiens

<400> 83
Lys Arg Gln Ile Tyr Val Ala Ala Phe
1               5


<210> 84
<211> 9
<212> PRT
<213> Homo sapiens

<400> 84
Pro Ser Lys Ala Trp Gly Glu Val Lys
1               5


<210> 85

<211> 9
<212> PRT
<213> Homo sapiens

<400> 85
Lys Ala Trp Gly Glu Val Lys Arg Gln
1               5


<210> 86
<211> 9
<212> PRT
<213> Homo sapiens

<400> 86
Trp Lys Glu Phe Gly Leu Asp Ser Val
1               5


<210> 87
<211> 10
<212> PRT
<213> Homo sapiens

<400> 87
Gln Trp Lys Glu Phe Gly Leu Asp Ser Val
1               5                   10


<210> 88
<211> 10
<212> PRT
<213> Homo sapiens

<400> 88
Glu Phe Gly Leu Asp Ser Val Glu Leu Ala
1               5                   10


<210> 89
<211> 91
<212> PRT
<213> Homo sapiens

<400> 89
Glu Val Pro Gln Leu Thr Asp Leu Ser Phe Val Asp Ile Thr Asp Ser
1               5                   10                  15
Ser Ile Gly Leu Arg Trp Thr Pro Leu Asn Ser Ser Thr Ile Ile Gly
            20                  25                  30
Tyr Arg Ile Thr Val Val Ala Ala Gly Glu Gly Ile Pro Ile Phe Glu
        35                  40                  45
Asp Phe Val Asp Ser Ser Val Gly Tyr Tyr Thr Val Thr Gly Leu Glu
    50                  55                  60
Pro Gly Ile Asp Tyr Asp Ile Ser Val Ile Thr Leu Ile Asn Gly Gly
65                  70                  75                  80
Glu Ser Ala Pro Thr Thr Leu Thr Gln Gln Thr
                85                  90


<210> 90
<211> 147
<212> PRT
<213> Homo sapiens

<400> 90
Cys Thr Phe Asp Asn Leu Ser Pro Gly Leu Glu Tyr Asn Val Ser Val
1               5                   10                  15
Tyr Thr Val Lys Asp Asp Lys Glu Ser Val Pro Ile Ser Asp Thr Ile

                20                  25                  30
Ile Pro Glu Val Pro Gln Leu Thr Asp Leu Ser Phe Val Asp Ile Thr
        35                  40                  45
Asp Ser Ser Ile Gly Leu Arg Trp Thr Pro Leu Asn Ser Ser Thr Ile
    50                  55                  60
Ile Gly Tyr Arg Ile Thr Val Val Ala Ala Gly Glu Gly Ile Pro Ile
65                  70                  75                  80
Phe Glu Asp Phe Val Asp Ser Ser Val Gly Tyr Tyr Thr Val Thr Gly
                85                  90                  95
Leu Glu Pro Gly Ile Asp Tyr Asp Ile Ser Val Ile Thr Leu Ile Asn
            100                 105                 110
Gly Gly Glu Ser Ala Pro Thr Thr Leu Thr Gln Gln Thr Ala Val Pro
        115                 120                 125
Pro Pro Thr Asp Leu Arg Phe Thr Asn Ile Gly Pro Asp Thr Met Arg
    130                 135                 140
Val Thr Trp
145

<210> 91
<211> 2823
<212> DNA
<213> Homo sapiens

<400> 91
   ctgcactttt gataacctga gtcccggcct ggagtacaat gtcagtgttt acactgtcaa 60
   ggatgacaag gaaagtgtcc ctatctctga taccatcatc ccaggtaata gaaaataagc 120
   tgctatcctg agagtgacat tccaataaga gtggggatta gcatcttaat ccccagatgc 180
   ttaagggtgt caactatatt tgggatttaa ttccgatctc ccagctgcac tttccaaaac 240
   caagaagtca aagcagcgat ttggacaaaa tgcttgctgt taacactgct ttactgtctg 300
   tgcttcactg ggatgctgtg tgttgcagcg agtatgtaat ggagtggcag ccatggcttt 360
   aactctgtat tgtctgctca catggaagta tgactaaaac actgtcacgt gtctgtactc 420
   agtactgata ggctcaaagt aatatggtaa atgcatccca tcagtacatt tctgcccgat 480
   tttacaatcc atatcaattt ccaacagctg cctatttcat cttgcagttt caaatccttc 540
   tttttgaaaa ttggatttta aaaaaaagtt aagtaaaagt cacaccttca gggttgttct 600
   ttcttgtggc cttgaaagac aacattgcaa aggcctgtcc taaggatagg cttgtttgtc 660
   cattgggtta taacataatg aaagcattgg acagatcgtg tccccctttg gactcttcag 720
   tagaatgctt ttactaacgc taattacatg ttttgattat gaatgaacct aaaatagtgg 780
   caatggcctt aacctaggcc tgtctttcct cagcctgaat gtgcttttga atggcacatt 840
   tcacaccata cattcataat gcattagcgt tatggccatg atgttgtcat gagttttgta 900
   tgggagaaaa aaaatcaatt tatcacccat ttattatttt ttccggttgt tcatgcaagc 960
   ttattttcta ctaaaacagt tttggaatta ttaaaagcat tgctgatact tacttcagat 1020
   attatgtcta ggctctaaga atggtttcga catcctaaac agccatatga tttttaggaa 1080
   tctgaacagt tcaaattgta ccctttaagg atgttttcaa aatgtaaaaa atatatatat 1140
   atatatatat tccctaaaag aatattcctg tttattcttc tagggaagca aactgttcat 1200
   gatgcttagg aagtcttttc agagaattta aaacagattg catattacca tcattgcttt 1260
   aacattccac caattttact actagtaacc tgatatacac tgctttattt tttcctcttt 1320
   ttttccctct attttccttt tgcctccccc tccctttgct ttgtaactca atagaggtgc 1380
   cccaactcac tgacctaagc tttgttgata taaccgattc aagcatcggc ctgaggtgga 1440
   ccccgctaaa ctcttccacc attattgggt accgcatcac agtagttgcg gcaggagaag 1500
   gtatccctat ttttgaagat tttgtggact cctcagtagg atactacaca gtcacagggc 1560
   tggagccggg cattgactat gatatcagcg ttatcactct cattaatggc ggcgagagtg 1620
   cccctactac actgacacaa caaacgggtg aattttgaaa acttctgcgt ttgagacata 1680
   gatggtgttg catgctgcca ccagttactc cggttaaata tggatgtttc atgggggaag 1740
   tcagcaattg gccaaagatt cagataggtg gaattggggg gataaggaat caaatgcatc 1800
   tgctaaactg attggagaaa aacacatgca atatcttcag tacactctca tttaaaccac 1860
   aagtagatat aaagcctaga gaaatacaga tgtctgctct gttaaatata aaatagcaaa 1920
   tgttcattca atttgaagac ctagaatttt tcttcttaaa taccaaacac gaataccaaa 1980
   ttgcgtaagt accaattgat aagaatatat caccaaaatg taccatcatg ctcttccttc 2040
   taccctttga taaactctac catgctcctt ctttgtagct aaaaacccat caaaatttag 2100
   ggtagagtgg atgggcattg ttttgaggta ggagaaaagt aaacttggga ccattctagg 2160
   ttttgttgct gtcactaggt aaagaaacac ctctttaacc acagtctggg gacaagcatg 2220
   caacatttta aaggttctct gctgtgcatg ggaaaagaaa catgctgaga accaatttgc 2280
   atgaacatgt tcacttgtaa gtagaattca ctgaatggaa ctgtagctct agatatctca 2340
   catgggggga agtttaggac cctcttgtct ttttgtctgt gtgcatgtat ttctttgtaa 2400
   agtactgcta tgtttctctt tgctgtgtgg caacttaagc ctcttcggcc tgggataaaa 2460
   taatctgcag tggtattaat aatgtacata aagtcaacat atttgaaagt agattaaaat 2520
   cttttttaaa tatatcaatg atggcaaaaa ggttaaaggg ggcctaacag tactgtgtgt 2580
   agtgttttat ttttaacagt agtacactat aacttaaaat agacttagat tagactgttt 2640
   gcatgattat gattctgttt cctttatgca tgaaatattg attttacctt tccagctact 2700
   tcgttagctt taattttaaa atacattaac tgagtcttcc ttcttgttcg aaaccagctg 2760
   ttcctcctcc cactgacctg cgattcacca acattggtcc agacaccatg cgtgtcacct 2820
   ggg 2823

<210> 92
<211> 9
<212> PRT
<213> Homo sapiens

<400> 92
Thr Ile Ile Pro Glu Val Pro Gln Leu
1               5

<210> 93
<211> 10
<212> PRT
<213> Homo sapiens

<400> 93
Asp Thr Ile Ile Pro Glu Val Pro Gln Leu
1               5                   10

<210> 94
<211> 10
<212> PRT
<213> Homo sapiens

<400> 94
Glu Val Pro Gln Leu Thr Asp Leu Ser Phe
1               5                   10

<210> 95
<211> 8
<212> PRT
<213> Homo sapiens

<400> 95
Thr Pro Leu Asn Ser Ser Thr Ile
1               5

<210> 96
<211> 8
<212> PRT
<213> Homo sapiens

<400> 96
Ile Gly Leu Arg Trp Thr Pro Leu
1               5

<210> 97
<211> 9
<212> PRT
<213> Homo sapiens

<400> 97
Ser Ile Gly Leu Arg Trp Thr Pro Leu
1               5

<210> 98
<211> 9

<212> PRT
<213> Homo sapiens

<400> 98
Leu Asn Ser Ser Thr Ile Ile Gly Tyr
1               5


<210> 99
<211> 10
<212> PRT
<213> Homo sapiens

<400> 99
Pro Leu Asn Ser Ser Thr Ile Ile Gly Tyr
1               5                   10


<210> 100
<211> 9
<212> PRT
<213> Homo sapiens

<400> 100
Thr Pro Leu Asn Ser Ser Thr Ile Ile
1               5


<210> 101
<211> 8
<212> PRT
<213> Homo sapiens

<400> 101
Ile Gly Tyr Arg Ile Thr Val Val
1               5


<210> 102
<211> 9
<212> PRT
<213> Homo sapiens

<400> 102
Ile Ile Gly Tyr Arg Ile Thr Val Val
1               5


<210> 103
<211> 10
<212> PRT
<213> Homo sapiens

<400> 103
Thr Ile Ile Gly Tyr Arg Ile Thr Val Val
1               5                   10


<210> 104
<211> 9
<212> PRT
<213> Homo sapiens

<400> 104
Ile Gly Tyr Arg Ile Thr Val Val Ala
1               5

<210> 105
<211> 10
<212> PRT
<213> Homo sapiens

<400> 105
Ile Ile Gly Tyr Arg Ile Thr Val Val Ala
1               5                   10


<210> 106
<211> 10
<212> PRT
<213> Homo sapiens

<400> 106
Glu Leu Ala Gly Ile Gly Ile Leu Thr Val
1               5                   10


<210> 107
<211> 10
<212> PRT
<213> Homo sapiens

<400> 107
Glu Ala Ala Gly Ile Gly Ile Leu Thr Val
1               5                   10


<210> 108
<211> 10
<212> PRT
<213> Homo sapiens

<400> 108
Phe Leu Pro Ser Asp Tyr Phe Pro Ser Val
1               5                   10


<210> 109
<211> 9
<212> PRT
<213> Homo sapiens

<400> 109
Ala Glu Met Gly Lys Tyr Ser Phe Tyr
1               5


<210> 110
<211> 15
<212> PRT
<213> Homo sapiens

<400> 110
Ala Phe Ser Pro Gln Gly Met Pro Glu Gly Asp Leu Val Tyr Val
1               5                   10                  15


<210> 111
<211> 12
<212> PRT
<213> Homo sapiens

<400> 111
Asn Tyr Ala Arg Thr Glu Asp Phe Phe Lys Leu Glu
1               5                   10

<210> 112
<211> 20
<212> PRT
<213> Homo sapiens

<400> 112
Pro Glu Gly Asp Leu Val Tyr Val Asn Tyr Ala Arg Thr Glu Asp Phe
1               5                   10                  15
Phe Lys Leu Glu
            20


<210> 113
<211> 14
<212> PRT
<213> Homo sapiens

<400> 113
Asn Tyr Ala Arg Thr Glu Asp Phe Phe Lys Leu Glu Arg Asp
1               5                   10


<210> 114
<211> 22
<212> PRT
<213> Homo sapiens

<400> 114
Pro Glu Gly Asp Leu Val Tyr Val Asn Tyr Ala Arg Thr Glu Asp Phe
1               5                   10                  15
Phe Lys Leu Glu Arg Asp
            20


<210> 115
<211> 15
<212> PRT
<213> Homo sapiens

<400> 115
Asn Tyr Ala Arg Thr Glu Asp Phe Phe Lys Leu Glu Arg Asp Met
1               5                   10                  15


<210> 116
<211> 14
<212> PRT
<213> Homo sapiens

<400> 116
Ala Phe Ser Pro Gln Gly Met Pro Glu Gly Asp Leu Val Tyr
1               5                   10


<210> 117
<211> 16
<212> PRT
<213> Homo sapiens

<400> 117
Val Asn Tyr Ala Arg Thr Glu Asp Phe Phe Lys Leu Glu Arg Asp Met
1               5                   10                  15


<210> 118
<211> 17

<212> PRT
<213> Homo sapiens

<400> 118
Ala Phe Ser Pro Gln Gly Met Pro Glu Gly Asp Leu Val Tyr Val Asn
1               5                   10                  15
Tyr

<210> 119
<211> 13
<212> PRT
<213> Homo sapiens

<400> 119
Ala Arg Thr Glu Asp Phe Phe Lys Leu Glu Arg Asp Met
1               5                   10

<210> 120
<211> 21
<212> PRT
<213> Homo sapiens

<400> 120
Ala Phe Ser Pro Gln Gly Met Pro Glu Gly Asp Leu Val Tyr Val Asn
1               5                   10                  15
Tyr Ala Arg Thr Glu
            20

<210> 121
<211> 9
<212> PRT
<213> Homo sapiens

<400> 121
Asp Phe Phe Lys Leu Glu Arg Asp Met
1               5

<210> 122
<211> 17
<212> PRT
<213> Homo sapiens

<400> 122
Tyr Val Asn Tyr Ala Arg Thr Glu Asp Phe Phe Lys Leu Glu Arg Asp
1               5                   10                  15
Met

<210> 123
<211> 19
<212> PRT
<213> Homo sapiens

<400> 123
Gln Gly Met Pro Glu Gly Asp Leu Val Tyr Val Asn Tyr Ala Arg Thr
1               5                   10                  15
Glu Asp Phe

<210> 124
<211> 9

<212> PRT
<213> Homo sapiens

<400> 124
Asn Tyr Ala Arg Thr Glu Asp Phe Phe
1               5


<210> 125
<211> 17
<212> PRT
<213> Homo sapiens

<400> 125
Arg Gly Ile Ala Glu Ala Val Gly Leu Pro Ser Ile Pro Val His Pro
1               5                   10                  15
Ile


<210> 126
<211> 12
<212> PRT
<213> Homo sapiens

<400> 126
Ala Val Gly Leu Pro Ser Ile Pro Val His Pro Ile
1               5                   10


<210> 127
<211> 11
<212> PRT
<213> Homo sapiens

<400> 127
Val Gly Leu Pro Ser Ile Pro Val His Pro Ile
1               5                   10


<210> 128
<211> 10
<212> PRT
<213> Homo sapiens

<400> 128
Gly Leu Pro Ser Ile Pro Val His Pro Ile
1               5                   10


<210> 129
<211> 13
<212> PRT
<213> Homo sapiens

<400> 129
Gly Tyr Tyr Asp Ala Gln Lys Leu Leu Glu Lys Met Gly
1               5                   10


<210> 130
<211> 8
<212> PRT
<213> Homo sapiens

<400> 130
Gly Tyr Tyr Asp Ala Gln Lys Leu
1               5

<210> 131
<211> 25
<212> PRT
<213> Homo sapiens

<400> 131
Arg Gly Ile Ala Glu Ala Val Gly Leu Pro Ser Ile Pro Val His Pro
1               5                   10                  15
Ile Gly Tyr Tyr Asp Ala Gln Lys Leu
            20                  25


<210> 132
<211> 27
<212> PRT
<213> Homo sapiens

<400> 132
Arg Gly Ile Ala Glu Ala Val Gly Leu Pro Ser Ile Pro Val His Pro
1               5                   10                  15
Ile Gly Tyr Tyr Asp Ala Gln Lys Leu Leu Glu
            20                  25


<210> 133
<211> 22
<212> PRT
<213> Homo sapiens

<400> 133
Ala Val Gly Leu Pro Ser Ile Pro Val His Pro Ile Gly Tyr Tyr Asp
1               5                   10                  15
Ala Gln Lys Leu Leu Glu
            20


<210> 134
<211> 21
<212> PRT
<213> Homo sapiens

<400> 134
Val Gly Leu Pro Ser Ile Pro Val His Pro Ile Gly Tyr Tyr Asp Ala
1               5                   10                  15
Gln Lys Leu Leu Glu
            20


<210> 135
<211> 20
<212> PRT
<213> Homo sapiens

<400> 135
Gly Leu Pro Ser Ile Pro Val His Pro Ile Gly Tyr Tyr Asp Ala Gln
1               5                   10                  15
Lys Leu Leu Glu
            20


<210> 136
<211> 19
<212> PRT
<213> Homo sapiens

<400> 136

Arg Gly Ile Ala Glu Ala Val Gly Leu Pro Ser Ile Pro Val His Pro
1               5                   10                  15
Ile Gly Tyr

<210> 137
<211> 14
<212> PRT
<213> Homo sapiens

<400> 137
Ala Val Gly Leu Pro Ser Ile Pro Val His Pro Ile Gly Tyr
1               5                   10

<210> 138
<211> 13
<212> PRT
<213> Homo sapiens

<400> 138
Val Gly Leu Pro Ser Ile Pro Val His Pro Ile Gly Tyr
1               5                   10

<210> 139
<211> 12
<212> PRT
<213> Homo sapiens

<400> 139
Gly Leu Pro Ser Ile Pro Val His Pro Ile Gly Tyr
1               5                   10

<210> 140
<211> 24
<212> PRT
<213> Homo sapiens

<400> 140
Val Gly Leu Pro Ser Ile Pro Val His Pro Ile Gly Tyr Tyr Asp Ala
1               5                   10                  15
Gln Lys Leu Leu Glu Lys Met Gly
            20

<210> 141
<211> 23
<212> PRT
<213> Homo sapiens

<400> 141
Gly Leu Pro Ser Ile Pro Val His Pro Ile Gly Tyr Tyr Asp Ala Gln
1               5                   10                  15
Lys Leu Leu Glu Lys Met Gly
            20

<210> 142
<211> 11
<212> PRT
<213> Homo sapiens

<400> 142
Ser Ser Ile Glu Gly Asn Tyr Thr Leu Arg Val
1               5                   10

<210> 143
<211> 16
<212> PRT
<213> Homo sapiens

<400> 143
Ser Ser Ile Glu Gly Asn Tyr Thr Leu Arg Val Asp Cys Thr Pro Leu
1               5                   10                  15


<210> 144
<211> 17
<212> PRT
<213> Homo sapiens

<400> 144
Ser Ser Ile Glu Gly Asn Tyr Thr Leu Arg Val Asp Cys Thr Pro Leu
1               5                   10                  15
Met


<210> 145
<211> 9
<212> PRT
<213> Homo sapiens

<400> 145
Arg Val Asp Cys Thr Pro Leu Met Tyr
1               5


<210> 146
<211> 18
<212> PRT
<213> Homo sapiens

<400> 146
Ser Ser Ile Glu Gly Asn Tyr Thr Leu Arg Val Asp Cys Thr Pro Leu
1               5                   10                  15
Met Tyr


<210> 147
<211> 10
<212> PRT
<213> Homo sapiens

<400> 147
Ser Leu Val His Asn Leu Thr Lys Glu Leu
1               5                   10


<210> 148
<211> 12
<212> PRT
<213> Homo sapiens

<400> 148
Met Tyr Ser Leu Val His Asn Leu Thr Lys Glu Leu
1               5                   10


<210> 149
<211> 7

<212> PRT
<213> Homo sapiens

<400> 149
Leu Pro Asp Arg Pro Phe Tyr
1               5


<210> 150
<211> 11
<212> PRT
<213> Homo sapiens

<400> 150
Asp Pro Leu Gly Leu Pro Asp Arg Pro Phe Tyr
1               5                   10


<210> 151
<211> 12
<212> PRT
<213> Homo sapiens

<400> 151
Ile Asp Pro Leu Gly Leu Pro Asp Arg Pro Phe Tyr
1               5                   10


<210> 152
<211> 13
<212> PRT
<213> Homo sapiens

<400> 152
Phe Ile Asp Pro Leu Gly Leu Pro Asp Arg Pro Phe Tyr
1               5                   10


<210> 153
<211> 14
<212> PRT
<213> Homo sapiens

<400> 153
Ala Phe Ile Asp Pro Leu Gly Leu Pro Asp Arg Pro Phe Tyr
1               5                   10


<210> 154
<211> 15
<212> PRT
<213> Homo sapiens

<400> 154
Arg Ala Phe Ile Asp Pro Leu Gly Leu Pro Asp Arg Pro Phe Tyr
1               5                   10                  15


<210> 155
<211> 20
<212> PRT
<213> Homo sapiens

<400> 155
Phe Asp Lys Ser Asn Pro Ile Val Leu Arg Met Met Asn Asp Gln Leu
1               5                   10                  15
Met Phe Leu Glu
            20

<210> 156
<211> 16
<212> PRT
<213> Homo sapiens

<400> 156
Glu Arg Ala Phe Ile Asp Pro Leu Gly Leu Pro Asp Arg Pro Phe Tyr
1               5                   10                  15


<210> 157
<211> 19
<212> PRT
<213> Homo sapiens

<400> 157
Phe Asp Lys Ser Asn Pro Ile Val Leu Arg Met Met Asn Asp Gln Leu
1               5                   10                  15
Met Phe Leu


<210> 158
<211> 17
<212> PRT
<213> Homo sapiens

<400> 158
Leu Glu Arg Ala Phe Ile Asp Pro Leu Gly Leu Pro Asp Arg Pro Phe
1               5                   10                  15
Tyr


<210> 159
<211> 18
<212> PRT
<213> Homo sapiens

<400> 159
Phe Asp Lys Ser Asn Pro Ile Val Leu Arg Met Met Asn Asp Gln Leu
1               5                   10                  15
Met Phe